# EUROPEAN PATENT APPLICATION

(11) **EP 3 150 217 A1**
(43) Date of publication of application: **05.04.2017**
(21) Application number: 15800382.2
(22) Date of filing: 29.05.2015
(51) Int. Cl.: A61K 38/14, A61K 38/17, A61P 31/04, A61P 31/10

(54) **COMPOSITION FOR PREVENTING OR TREATING STAPHYLOCOCCUS AUREUS INFECTION**

(30) Priority: 29.05.2014 US 201462004564 P; 22.08.2014 US 201462040452 P; 30.08.2014 US 201462044196 P
(71) Applicant: Green Cross Corporation, Giheung-gu, Yongin-si Gyeonggi-do 446-770 (KR); Pusan National University Industry-University Cooperation Foundation, Busan 609-735 (KR)
(72) Inventor: LEE, Bok Luel, Busan 609-310 (KR); LEE, Min Ja, Yangsan-si Gyeongsangnam-do 626-250 (KR); LEE, Jong Ho, Jeonju-si Jeollabuk-do 560-295 (KR); SEONG, Min Young, Busan 602-808 (KR); AHN, Dong Ho, Yongin-si Gyeonggi-do 446-770 (KR); TAKAHASHI, Kazue, Cambridge, Massachusetts 02139 (US); KUROKAWA, Kenji, Sasebo Nagasaki 859-3215 (JP)
(74) Representative: Rowe, Daniel
(86) International application number: PCT/KR2015/005431
(87) International publication number: WO 2015/183041

(57) **Abstract**

A composition containing wall teichoic acid-attached peptidoglycan (WTA-PGN) as an active ingredient, a method for preventing or treating *Staphylococcus aureus* infectious diseases using the composition, and a method for preparing a soluble WTA-PGN which can be used as an active ingredient in the composition are provided. The composition of the present invention can be effectively used for preventing or treating *Staphylococcus aureus* infectious diseases by opsonophagocytosis due to antigen-antibody reaction and neutrophil-mediated phagocytosis due to T cell activation at the early stage of infection.

## Description

### Technical Field

The present invention relates to a composition for preventing or treating *Staphylococcus aureus* infectious diseases, and more specifically, to a composition comprising a wall teichoic acid-attached peptidoglycan (WTA-PGN, hereinafter) as an active ingredient, a method for preventing or treating *Staphylococcus aureus* infectious diseases using the composition, and a method for preparing a soluble WTA-PGN which can be used as an active ingredient in the composition.

### Background Art

*Staphylococcus aureus* can cause severe infections in human skin, soft tissue, and bloodstream (Lowy FD, The New England journal of medicine, 339: 520 - 532, 1998). Additionally, *Staphylococcus aureus* can be modified into a methicillin-resistant *Staphylococcus aureus* (MRSA) strain which is resistant to methicillin, a beta-lactam antibiotic, and such MRSA infection is difficult to treat and has a poor prognosis thus becomes a big social issue. In particular, community-associated MRSA (CA-MRSA) has been appearing in children, etc., who had not been hospitalized, making the treatment more difficult along with the conventional hospital-associated MRSA (HA-MRSA). The *USA300* MRSA strain spreading in the U.S. recently has been inducing serious diseases in children or immunosuppressed people, and thus there is a need for the development of a novel vaccine having the effects of prevention and treatment against MRSA infections.

Although the vaccine candidate for *Staphylococcus aureus* developed by foreign researchers showed good prognosis in clinical tests, they all failed to pass the clinical tests, and thus a clinically effective vaccine for *Staphylococcus aureus* has not been developed until now.

Recent studies suggested that, both humoral and cellular immunity are necessary to function as a *Staphylococcus aureus* vaccine, where the humoral immunity accelerates opsonophagocytosis by producing serum antibodies specific to particular vaccine candidate materials and the cellular immunity recruits neutrophils and accelerates phagocytosis by producing T cell-mediated IL-17A at the early stage of MRSA infection, and that the acceleration of the phagocytic cell effector function by T cell activation can provide a protection from *Staphylococcus aureus* infection. Additionally, foreign researchers reported that the *Staphylococcus aureus* infection can increase the number of memory γδ-T cells in a mouse model thereby exhibiting a protective effect against the infection (J. Immunol., 192 (8): 3697 - 3708, 2014). This result suggests that the exposure to *Staphylococcus aureus* induces γδ-T cells' memory responses in a manner similar to αβ T cells and the induction of memory γδ-T cells producing IL-17A can exhibit a strong host-protecting effect against *Staphylococcus aureus.* However, it has not been known until now regarding what material of *Staphylococcus aureus* exhibits such protective effect.

Unlike gram negative bacteria, the cell wall of gram positive bacteria such as *Staphylococcus aureus* largely consists of four kinds of components including peptidoglycan (PGN), wall teichoic acid (WTA), lipoteichoic acid (LTA), and capsular polysaccharide (CP).

Rapid recognition of invading pathogens by biodefense proteins of a host, and rapid removal of the pathogens by selective activation of innate immunity such as a complement system, are very important reactions. However, the ligand materials of bacteria recognized by the *in-vivo* defense proteins in a host have not been clearly identified and thus there is a difficulty in preventing and treating infectious diseases caused by pathogens.

In particular, ligands such as WTA, LTA, PGN, CP and lipoprotein, which are cell wall components of *Staphylococcus aureus,* are mostly glycopolymers with complex structures, and are purified along with other materials thus is difficult to be isolated/purified as a single material. Additionally, since various kinds of cell wall components are exposed to the outside, it is not easy to identify which of the components may act as a ligand of the *in-vivo* defense proteins of a host.

The inventors of the present invention have isolated WTA-PGN from a particular mutated strain of *Staphylococcus aureus* and have confirmed that the WTA-PGN can function as a ligand of the *in-vivo* defense proteins of a host capable of inducing early *in-vivo* immune responses against *Staphylococcus aureus* infection, thus is effective for the prevention and treatment of *Staphylococcus aureus* infectious diseases.

### Disclosure of Invention

### Technical Problem

Therefore, an object of the present invention is to provide a composition for preventing or treating *Staphylococcus aureus* infectious diseases.

It is another object of the present invention to provide a method for preventing or treating *Staphylococcus aureus* infectious diseases using the composition.

It is still another object of the present invention to provide a method for preparing a soluble WTA-PGN which can be used as an active ingredient in the composition.

### Solution to Problem

In order to achieve the above objects, the present invention provides a composition for preventing or treating *Staphylococcus aureus* infectious diseases comprising WTA-PGN as an active ingredient.

Also, the present invention provides a method for preventing or treating *Staphylococcus aureus* infectious diseases comprising administering the above-mentioned composition to a subject in need thereof.

Further, the present invention provides a method for preparing a soluble WTA-PGN, comprising the steps of:
(1) obtaining a strain with a double mutation in which lipoprotein diacylglycerol transferase (*lgt*) and *O*-acetyl transferase *(oatA)* genes are deleted from a wild-type *Staphylococcus aureus;*
(2) disrupting the strain with a double mutation and obtaining an insoluble WTA-PGN from the disrupted strain;
(3) treating the insoluble WTA-PGN with a β-lytic enzyme;
(4) obtaining a fraction comprising a soluble WTA-PGN from the enzyme-treated product in Step (3);
(5) treating the fraction comprising a soluble WTA-PGN with a lysozyme or a mutanolysin; and
(6) obtaining a soluble WTA-PGN from the enzyme-treated product in Step (5).

### Brief Description of Drawings

The above objects and features of the present invention will become apparent from the following description of the invention, when taken in conjunction with the accompanying drawings, which respectively show:

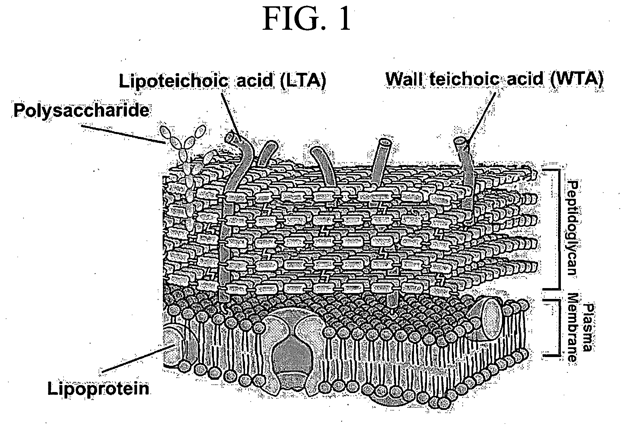

FIG. 1 is a schematic diagram illustrating the cell wall structure of *Staphylococcus aureus.*
FIG. 2 is a flow chart illustrating the respective processes for obtaining a soluble WTA-PGN, a soluble WTA, and a soluble PGN from a strain with RN4220 *Δlgt*/*Δoat* mutation.
FIG. 3 shows an elution pattern of insoluble WTA-PGN separated by HiTrap-Q column after treating the insoluble WTA-PGN with β-lytic enzyme (A) and gel mobility of the eluted fraction (B); and an elution pattern of WTA-PGN separated by HiTrap-Q column after treating β-lytic enzyme-treated WTA-PGN with lysozyme (C), and gel mobility (D).
FIG. 4 shows an elution pattern of WTA-PGN separated by Sephacryl S-200 HR column (A) and gel mobility (B); and the result of quantitation of the amount of IL-17A production (C) at the time of an intraperitoneal injection of WTA-PGN in a mouse.
FIG. 5 shows an elution pattern of WTA-PGN separated by the 1^{st} reverse phase column (5A) and gel mobility (5B); an elution pattern of WTA-PGN separated by the 2^{nd} reverse phase column (5C), and gel mobility (5D); and the result of quantitation of the amount of IL-17A production (5E) induced at the time of an intraperitoneal injection of WTA-PGN in a mouse.
FIG. 6 shows an elution pattern of WTA-PGN separated by HiTrap-Q column after treating insoluble WTA-PGN with trichloroacetic acid (TCA) (A) and gel mobility (B).
FIG. 7 shows an elution pattern of soluble PGN separated by Hitrap-Q column.
FIG. 8 shows an elution pattern of soluble PGN separated by Toyopearl HW 55 S column.
FIG. 9 shows 27% PAGE and silver nitrate staining (A), silica gel thin layer chromatography (B), and the amount of residues of phosphate and GlcNAc (C), of WTA-PGN and WTA, respectively.

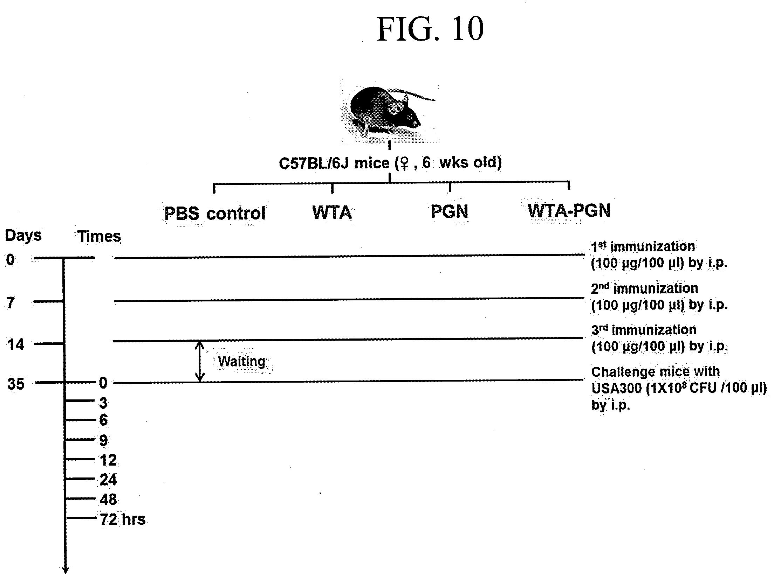

FIG. 10 shows time schedule for 3 times of intraperitoneal immunization of WTA, PGN, and WTA-PGN; and MRSA (*USA300*) infection for the observation of a γδ T cell-induced initial immune response (cellular immunity) and a memory immune response.
FIG. 11 shows the production of IL-17A (11A) and IL-1β (11B) in a mouse induced by intraperitoneal injection of each of PBS, WTA-PGN, and a mixture of WTA and PGN, according to various concentrations and time.
FIG. 12 shows the production of IL-17A, IL-1β, and IL-10 over time after the injection of WTA-PGN.
FIG. 13 shows the distribution rate of γδ T cells capable of producing IL-17A after the injections of PBS and WTA-PGN analyzed by flow cytometry analysis.
FIG. 14 shows IL-17A production mediated by CD4⁺- and CD8⁺ T cells after the injections of PBS and WTA-PGN.
FIG. 15 shows the IL-17A production (15A) and the IL-1β production (15B) in wild-type and Vγ2/4^{-/-} mice treated with PBS, WTA-PGN, and a mixture of WTA and PGN.
FIG. 16 shows the production of IL-17A (16A), IL-1β (16B), IL-23 (16C), IFNγ (16D), and IL-10 (16E) induced by the infection of *USA300* strain, in each mouse pretreated with PBS, WTA, PGN, and WTA-PGN.
FIG. 17 shows the expression of CD44 and CD27, the memory γδ T cell markers, in γδ T cells of a mouse pretreated with WTA-PGN (17A); and the expression level of IL-17A in these memory γδ T cells (17B).
FIG. 18 shows the IL-17A expression in memory γδ T cells in each mouse pretreated with PBS, WTA-PGN, PGN, and WTA, which are the amount of intracellular IL-17A production measured by FACS (18A) and the amount of extracellular IL-17A production measured by ELISA (18B).
FIG. 19 shows the expression of IL-17A in CD4⁺-, CD8⁺-, and γδ T cells after pretreatment with WTA-PGN.
FIG. 20 shows the differentiation results of the subset population or γδ TCR at the time of producing memory γδ T cells.
FIG. 21 shows the IL-23 expression in dendritic cells in a mouse pretreated with WTA-PGN and a mouse not pretreated with WTA-PGN.
FIG. 22 shows the images of organ shapes and abscess formation within the peritoneum of mice infected with *USA300* strain after pretreatment with PBS, WTA, PGN, and WTA-PGN.
FIG. 23 shows the survival of a mouse, in which memory γδ T cells were induced by immunization with WTA-PGN, after the *USA300* infection.
FIG. 24 shows the shapes (24A) and volume (24B) of abscess in mice infected with methicillin-sensitive *Staphylococcus aureus* (MSSA) (*S. aureus* NRS184 strain) after immunization with WTA-PGN.
FIG. 25 shows the absence/presence of formation of dermal abscess (25A), dermonecrosis area (25B), and abscess volume (25C) in NZW rabbits infected with MRSA after immunization with WTA-PGN.
FIG. 26 shows the absence/presence of formation of dermal abscess, dermonecrosis area and abscess volume in NZW rabbits (A) and guinea pigs (B) after immunization with WTA-PGN.
FIG. 27 shows the images of abscess and hemolysis in the peritoneum of guinea pigs immunized with WTA-PGN after MRSA infection.
FIG. 28 shows the production of IL-17A (28A) and IL-1β (28B) in wild-type mice, mice with a TLR-9 gene defect, and mice with a Caspase-1 gene defect each immunized with WTA-PGN.
FIG. 29 shows the expression of IL-17A (29A), IL-1β (29B), IL-23 (29C), and IFNγ (29D) in macrophages, dendritic cells, and γδ T cells of mice obtained after pretreatment with WTA-PGN.
FIG. 30 shows the gene expression features (30A to 30J), in wild-type mice and mice with a NLRP3 gene defect, induced by WTA-PGN injection.
FIG. 31 shows the time schedule of experiments in which each mouse, immunized with WTA, PGN, and WTA-PGN, respectively, was infected with MRSA USA 300 to its caudal vein for the observation of humoral immune responses by anti-IgG production.
FIG. 32 shows the amount of anti-IgG production in mice immunized with WTA and WTA-PGN, respectively.
FIG. 33 shows the change in body weight of mice immunized with WTA, PGN, and WTA-PGN, respectively, after *USA300* cell infection for a week.
FIG. 34 shows the images of the kidneys of mice immunized with WTA, PGN, and WTA-PGN, respectively, and bacterial loads (CFU). FIG. 34A represents images of the kidneys of mice immunized with (i) PBS, (ii) WTA, and (iii) WTA-PGN, respectively; and FIG. 34B represents the CFU of MRSA strain present in the kidneys (CFU/g in the kidneys) of mice immunized with (i) PBS, (ii) WTA, and (iii) WTA-PGN, respectively.
FIG. 35 shows the histopathological images in the kidneys of mice infected with MRSA (*USA300*) via bloodstream after immunization with WTA, PGN, and WTA-PGN, respectively.

### Mode for the Invention

Hereinafter, the present invention is explained in detail by Examples. The following Examples are intended to further illustrate the present invention without limiting its scope.

Herein below, the terms used in the present invention are defined.

As used herein, the term "wall teichoic acid (WTA)", being one of the cell wall components of *Staphylococcus aureus* (*S. aureus*), refers to a sugar polymer consisting of *N*-acetylmannosamine-(β-1,3)-*N-*acetylglucosamine along with a glycerol phosphate repeating unit and a ribitol phosphate repeating unit.

As used herein, the term "peptidoglycan" refers to a repeating sugar polymer of *N-*acetylmuramate (MurNAc) and *N*-acetylglucosamine (GlcNAc) linked by a bond between stem peptides.

As used herein, the term "wall teichoic acid-attached peptidoglycan (WTA-PGN)" refers to a structure in which wall teichoic acid and peptidoglycan are covalently linked, and in the present invention, it is used interchangeably with "WTA-PGN".

The present invention provides a composition for preventing or treating *Staphylococcus aureus* infectious diseases containing WTA-PGN as an active ingredient.

In an exemplary embodiment of the present invention, the WTA-PGN may be represented by General Formula 1 below: 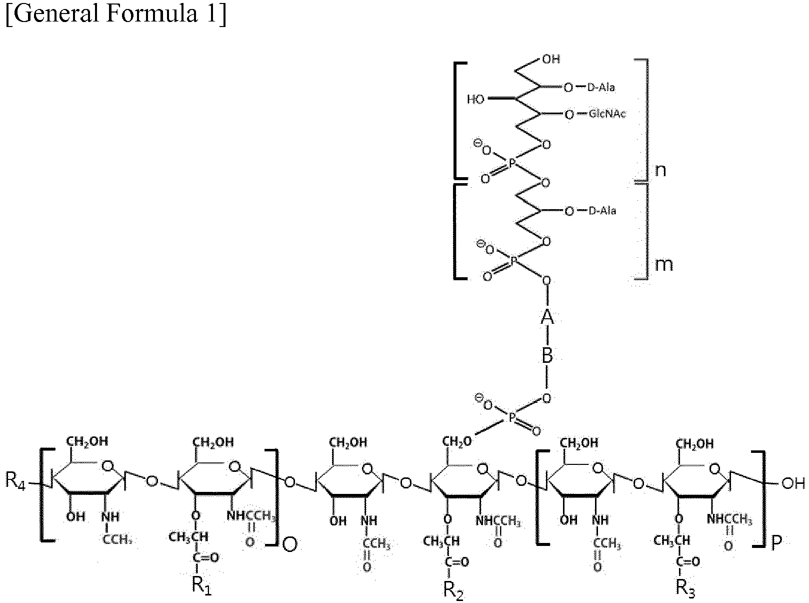 wherein, in General Formula 1, n is an integer of 10 to 50; m is an integer of 1 to 3; A is *N*-acetylmannosamine (ManNAc); B is *N*-acetylglucosamine (GlcNAc); O and P are each independently an integer of 0 to 5; R₁ to R₃ are each independently hydroxy, tetrapeptide or pentapeptide; and R₄ is hydroxy or *N*-acetylmuramic acid (MurNAc).

In another exemplary embodiment of the present invention, the WTA-PGN may be represented by General Formula 1 above, wherein n is an integer of 10 to 50; m is an integer of 1 to 3; A is *N*-acetylmannosamine (ManNAc); B is *N*-acetylglucosamine (GlcNAc); O and P are each independently an integer of 0 to 5; R₁ to R₃ are each independently hydroxy, tetrapeptide or pentapeptide; R₄ is hydroxy or *N*-acetylmuramic acid (MurNAc); and A and B are connected by a β-position with each other.

In still another exemplary embodiment of the present invention, the WTA-PGN may be represented by General Formula 1 above, wherein n is an integer of 35 to 45; m is 3; A is *N*-acetylmannosamine (ManNAc); B is *N*-acetylglucosamine (GlcNAc); O and P are each independently an integer of 0 to **3;** R₁ to R₃ are each independently hydroxy, tetrapeptide or pentapeptide; and R₄ is hydroxy or *N*-acetylmuramic acid (MurNAc).

In still another exemplary embodiment of the present invention, the WTA-PGN may be represented by General Formula 1 above, wherein n is 40; m is 3; A is *N-*acetylmannosamine (ManNAc); B is *N*-acetylglucosamine (GlcNAc); O and P are each independently an integer of 0 to 5; R₁ and R₂ are each independently tetrapeptide; R₃ is hydroxy, tetrapeptide or pentapeptide; and R₄ is hydroxy or *N*-acetylmuramic acid (MurNAc).

In still another exemplary embodiment of the present invention, the WTA-PGN may be represented by General Formula 1 above, wherein n is 40; m is 3; A is *N-*acetylmannosamine (ManNAc); B is *N*-acetylglucosamine (GlcNAc); O and P are each independently an integer of 0 to 5; R₁ and R₂ are each independently tetrapeptide; R₃ is hydroxy, tetrapeptide or pentapeptide, wherein the tetrapeptide is -A₁-A₂-A₃-A₄, in which A₁ is Ala or Gly, A₂ is Glu or Asp, A₃ is Lys, Arg or His; and A₄ is Ala or Gly; and R₄ is hydroxy or *N*-acetylmuramic acid (MurNAc).

In still another exemplary embodiment of the present invention, the WTA-PGN may be represented by General Formula 1 above, wherein n is 40; m is 3; A is *N-*acetylmannosamine (ManNAc); B is *N*-acetylglucosamine (GlcNAc); O and P are each independently an integer of 0 to 5; R₁ and R₂ are each independently tetrapeptide; R₃ is hydroxy, tetrapeptide or pentapeptide, wherein the tetrapeptide is -(L-Ala)-(*D*-Glu)-(*L-*Lys)-(D-Ala); and R₄ is hydroxy or *N*-acetylmuramic acid (MurNAc).

In still another exemplary embodiment of the present invention, any of R₁ to R₃ of the WTA-PGN may form a crosslinking with any of R₁ to R₃ of another WTA-PGN, and accordingly, the WTA-PGN may be present in the form of a dimer in which two WTA-PGNs are linked together.

The composition according to the present invention may be used for preventing or treating *Staphylococcus aureus* infectious diseases, and the *Staphylococcus aureus* inducing *Staphylococcus aureus* infectious diseases may be methicillin-resistant *Staphylococcus aureus* (MRSA), methicillin-sensitive *Staphylococcus aureus* (MSSA), or pathogenic *Staphylococcus aureus.*

Examples of the *Staphylococcus aureus* infectious diseases may include soft tissue infection, pyogenic arthritis, pyogenic osteomyelitis, otitis media, pneumonia, sepsis, acute respiratory tract infection, catheter-related infection, postoperative infection, bacteremia, endocarditis, and food poisoning, but are not limited thereto.

The composition according to the present invention may further comprise a pharmaceutically acceptable carrier, diluent, and/or adjuvant, in addition to the WTA-PGN.

The composition according to the present invention may further contain a pharmaceutically acceptable carrier, diluent, and/or adjuvant.

The carrier used in the composition according to the present invention may be determined based on the administration method and route, and the standard drug composition. For example, the carrier may be a carrier protein (*i.e.,* bovine serum albumin (BSA), ovalbumin (OVA), human serum albumin (HSA), and keyhole limpet hemocyanin (KLH)), a solubilizer (*i.e.,* ethanol, polysorbate, and Cremophor EL™), an isotonic agent, a preservative, an antioxidant, an excipient (*i.e.,* lactose, starch, crystalline cellulose, mannitol, maltose, calcium hydrogen phosphate, light anhydrous silicic acid, and calcium carbonate), a binder (*i.e.,* starch, polyvinylpyrrolidone, hydroxypropyl cellulose, ethyl cellulose, carboxymethyl cellulose, and gum Arabic), a lubricant (*i.e.,* magnesium stearate, talc, hardened oil, etc.), and a solubilizer (*i.e.,* lactose, mannitol, maltose, polysorbate, macrosol, polyoxyethylene, and hardened castor oil). If necessary, glycerin, dimethylacetamide, 70% sodium lactate, a surfactant or basic material (*i.e.,* sodium hydroxide, ethylenediamine, ethanol amine, sodium bicarbonate, alginine, Meglumine™, and trisaminomethane) may be contained. Specifically, for enhancing the antigenicity, the vaccine composition according to the present invention may be combined with a known KLH solution (Calbiotec, 125 mg/mL 50% glycerol solution) as a carrier protein.

The diluent used in the composition according to the present invention is selected based on the administration method and route, and actual standard drug composition. Examples of the diluent may include water, saline, phosphate buffered saline, and a bicarbonate solution.

The adjuvant used in the composition according to the present invention is selected based on the administration method and route, and actual standard drug composition. Examples of the adjuvant may include cholera toxin, heat-labile endotoxin (LT) of *E. coli,* liposome, and immune stimulating complexes (ISCOM).

The administration route may vary according to the age, weight, sex, and general health conditions of a subject to be administered having the risk of *Staphylococcus aureus* infection. However, the administration may be performed by any of the oral administration and parenteral administration (e.g., intravenous administration, arterial administration, and topical administration), and, preferably, the parenteral administration.

Formulation types for oral and parenteral administration and preparation methods thereof are known to those of ordinary skill in the art. The formulation types for oral and parenteral administration can be prepared by a conventional process, for example, by mixing with a pharmaceutically acceptable carrier described above. Examples of the formulation types for oral administration may include solid or liquid formulations such as solvents, tablets, granules, powdered drugs or capsules. Examples of the formulation types for parenteral administration may include solvents, suspensions, ointments, creams, suppositories, eye drops, nasal drops, and ear drops. For the sustained release of the formulations of the present invention, a biodegradable polymer (e.g., poly-*D,L*-lactid-co-glycolide or polyglycolide) may be added to a bulk base (for references, *see* U.S. Patent Nos. 5,417,986, 4,675,381, and 4,450,150). For oral administration, a flavoring agent and a coloring agent may be added. Suitable pharmaceutical carriers, diluents, and pharmaceutically necessary materials for their use are described in Remington's Pharmaceutical Sciences.

The administration dose of the composition according to the present invention may be determined based on the kinds of adjuvants, administration method and frequency, and desired effects, and generally, the dose may be in an amount of 1 µg to 100 mg of WTA per each administration for adults. When an adjuvant is added to the composition of the present invention, the dose may be generally in an amount of 1 µg to 1 mg of WTA per each administration for adults. The administration may be conducted several times if necessary. For example, after the initial administration of the composition at regular intervals, the composition may be administered again three times for supplementary purposes. Selectively, the composition for the first and second supplementation may be administered using the same formulation, respectively, from the 8^{th} week to the 12^{th} week and from the 16^{th} week to the 20^{th} week.

Additionally, the present invention provides a method for preventing or treating *Staphylococcus aureus* infectious diseases in a subject comprising administering the above-mentioned composition to a subject in need thereof.

As described above, the *Staphylococcus aureus* infectious diseases can be prevented or treated by administering the composition according to the present invention to thereby simultaneously induce opsonophagocytosis and phagocytosis in a subject.

The method of the present invention can increase the number of γδ-T cells, the amount of IL-17A production, and the amount of IL-1β production in a subject within 24 hours after the administration of the composition. Additionally, the method of the present invention can increase the amount of IL-10 production in the subject 12 hours after the administration of the composition.

Furthermore, the present invention provides a method for preparing a soluble wall teichoic acid-attached peptidoglycan (WTA-PGN), comprising :
(1) obtaining a double mutant strain in which lipoprotein diacylglycerol transferase (*lgt*) and *O*-acetyl transferase *(oatA)* genes are deleted from a wild-type *Staphylococcus aureus;*
(2) disrupting the strain with a double mutation and obtaining an insoluble WTA-PGN from the disrupted strain;
(3) treating the insoluble WTA-PGN with a β-lytic enzyme;
(4) obtaining a fraction containing a soluble WTA-PGN from the enzyme-treated product in Step (3);
(5) treating the fraction containing a soluble WTA-PGN with a lysozyme or a mutanolysin; and
(6) obtaining a soluble WTA-PGN from the enzyme-treated product in Step (5).

Hereinafter, the method for preparing the soluble WTA-PGN according to the present invention will be described in detail.

In the method of the present invention, in Step (1), a strain with a double mutation in which lipoprotein diacylglycerol transferase *(lgt)* and O-acetyl transferase (*oatA*) genes are deleted from wild-type *Staphylococcus aureus* is obtained.

The double mutant strain obtained in Step (1), *Δlgt4oatA,* in which lipoprotein diacylglycerol transferase *(lgt)* and O-acetyl transferase *(oatA)* genes were deleted, has little possibility of lipoprotein contamination due to the deletion of *lgt* gene, and thus a purer WTA-PGN can be easily obtained from the same. Additionally, the double mutant strain used in in Step (1) has no acetyl group in the MurNAc residue of PGN due to the deletion of *oatA* gene and thus the WTA-PGN produced above can be easily decomposed by lysostaphin or β-lytic enzyme in Step (2).

The double mutant strain may be obtained by a conventional mutagenesis known traditionally, from a wild-type *Staphylococcus aureus,* e.g., methicillin-resistant *Staphylococcus aureus* (MRSA), methicillin-sensitive *Staphylococcus aureus* (MSSA) or pathogenic *Staphylococcus aureus.* For example, the double mutant strain may be prepared by transforming a T363 strain (Nakayama M et al., Journal of Immunology 189: 5903 - 591, 2012), which has a deletion of lipoprotein diacylglycerol transferase *(lgt)* gene, and a T0003 strain (Park KH et al., Journal of Biological Chemistry 285, 27167 - 27175, 2010), which has erythromycin resistance and has a deletion of *O-*acetyl transferase *(oatA)* gene, using phage 80 as a mediator.

In the method of the present invention, in Step (2), the double mutant strain is disrupted, and insoluble WTA-PGN is obtained from the disrupted product.

Step (2) may be performed referring to the method described in the reference [Park KH et al., Journal of Biological Chemistry 285, 27167 - 27175, 2010; Jung DJ et al., Journal of Immunology 2012, 189: 4951 - 4959, 2012]

For example, Step (2) may comprise culturing the double mutant strain obtained in Step (1), disrupting cells, and obtaining insoluble WTA-PGN from the resultant.

In the method of the present invention, in Step (3), the insoluble WTA-PGN is treated with β-lytic enzyme.

The β-lytic enzyme plays the role of decomposing pentaglycine ((Gly)₅) bridge that connects a stempeptide present in the MurNAc residue of the insoluble WTA-PGN obtained in Step (2) thereby converting the insoluble WTA-PGN into soluble WTA-PGN.

The β-lytic enzyme is commercially available or may be isolated and purified according to the method described in the reference [Li et al,. Journal of Biochemistry 122, 772 - 778, 1997]. Examples of the β-lytic enzyme may include lysostaphin, but is not limited thereto.

Step (3) may be performed by suspending the insoluble WTA-PGN obtained in Step (2) in a buffer solution, adding with β-lytic enzyme and reacting them while stirring at a temperature of 30 to 40°C for 10 hours to 14 hours.

In the method of the present invention, in Step (4), a fraction containing the soluble WTA-PGN is obtained from the enzyme-treated product of Step (3).

In the above Step, the β-lytic enzyme-treated product is passed through high performance liquid chromatography (HPLC) to provide fractions and the fraction containing the soluble WTA-PGN is selected from the fractions.

The fraction containing the soluble WTA-PGN can be obtained by passing the enzyme-treated product of Step (3) through HPLC. Among the fractions which are obtained by passing through HPLC, the fraction containing the soluble WTA-PGN can be confirmed by PAGE or silver nitrate staining.

Examples of the column to be used in the HPLC purification may include HiTrap-Q (GE Healthcare), which is an anion exchange resin that binds to the anions of ribitol phosphate of WTA, but is not limited thereto.

In the method of the present invention, in Step (5), the fraction containing the soluble WTA-PGN is treated with lysozyme or mutanolysin.

The lysozyme or mutanolysin used above can decompose the binding between MurNAc and GlcNAc of PGN in the WTA-PGN thereby converting the polymeric PGN into an oligomeric PGN.

Step (5) may be performed by suspending the soluble WTA-PGN obtained in Step (3) in a buffer solution, adding with lysozyme or mutanolysin and stirring at 30°C to 40°C for 10 hours to 14 hours.

In the method of the present invention, in Step (6), a soluble WTA-PGN is obtained from the enzyme-treated product of Step (5).

The soluble WTA-PGN can be obtained by passing the enzyme-treated product of Step (5) through HPLC.

In the above Step, the lysozyme or mutanolysin enzyme-treated product is passed through HPLC to provide fractions and the fraction containing soluble WTA-PGN is selected from the above fractions. The selection of the above fraction may be performed based on the amount of IL-17A produced after the intraperitoneal injection of each fraction into a mouse.

Examples of the column to be used in the HPLC purification may include HiTrap-Q (GE Healthcare), but are not limited thereto.

The method for preparing the soluble WTA-PGN according to the present invention may further include an additional purification of WTA-PGN after Step (6).

The additional purification of WTA-PGN may be performed by gel filtration chromatography or reverse phase liquid chromatography.

In an exemplary embodiment of the present invention, the soluble WTA-PGN prepared in Step (6) may be purified further by gel filtration chromatography using Sephacryl S-200 HR column or reverse phase liquid chromatography using Symmetry Shield™ RP18 column.

In another exemplary embodiment of the present invention, the soluble WTA-PGN prepared in Step (6) may be purified further by gel filtration chromatography using Sephacryl S-200 HR column and two times of reverse phase liquid chromatography using Symmetry Shield™ RP18 column.

The fraction(s) which passed through the chromatography may be selected based on the amount of IL-17A produced after the intraperitoneal injection into a mouse.

The present invention is further described and illustrated in examples provided below, which are, however, not intended to limit the scope of the present invention.

### Example 1: Preparation of a strain for obtaining WTA derivatives

For the isolation of WTA-PGN, *S. aureus* T384 strain (RN4220 *ΔlgtΔoatA* double mutant) was prepared according to the method described in the reference [Kazue Takahashi et al., Plos One 8: e69739, 2013].

In brief, *S. aureus* T384 strain was prepared by transforming a T363 strain (Nakayama M et al., Journal of Immunology 189: 5903 - 591, 2012), which has a deletion of lipoprotein diacylglycerol transferase (*lgt)* gene, and a T0003 strain (Park KH et al., Journal of Biological Chemistry 285, 27167 - 27175, 2010), which has erythromycin resistance and has a deletion of *O*-acetyl transferase (*oatA*) gene, using phage 80 as a mediator. The strain can be used for the isolation of WTA, WTA-PGN, and PGN without lipoprotein contamination due to the deletion of *lgt* gene, and the isolated PGN can be easily decomposed by lysozyme due to the absence of an acetyl group in the oxygen at the position of PGN MurNac residue 6 due to the deletion of *oatA* gene.

### Example 2: Isolation and purification of soluble WTA-PGN

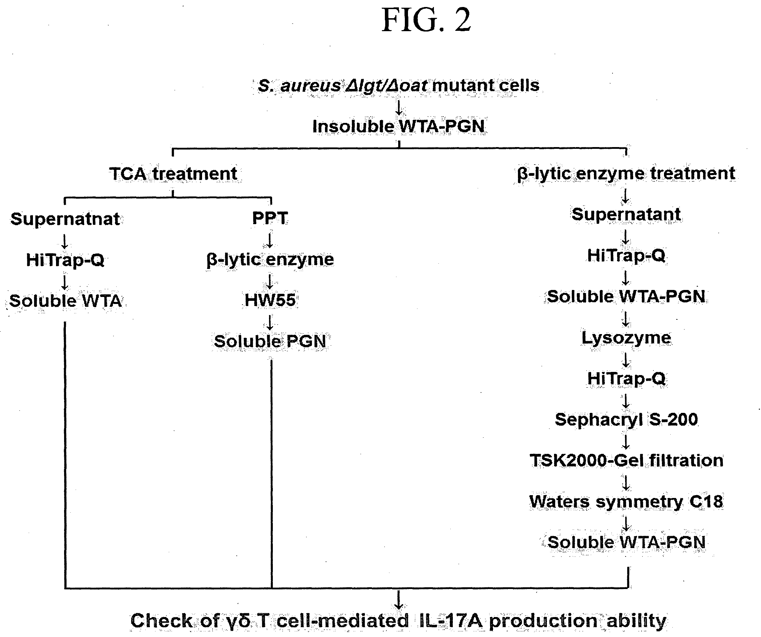

Insoluble WTA-PGN was obtained from the *Δlgt*/*ΔoatA* mutant strain prepared in Example 1 and soluble WTA was isolated from the insoluble WTA-PGN and purified (*see* FIG. 2).

### <2-1> Isolation and purification of insoluble WTA-PGN derivatives

The insoluble WTA-PGN was isolated and purified by modifying the method described in the reference [Park KH et al., Journal of Biological Chemistry 285, 27167 - 27175, 2010; Jung DJ et al., Journal of Immunology 2012, 189: 4951 - 4959, 2012].

Specifically, the *ΔlgtΔoatA* mutant strain of Example 1 was cultured using an incubator and the resulting bacterial cells were recovered. The recovered bacterial cells (10 mL) were suspended in 20 mM citrate buffer (pH 4.5; 30 mL), and 50 µL of them was diluted 400 fold and adjusted to have an OD₆₀₀ₙₘ of 0.8 using a spectrophotometer. Then, to remove 20 mM citrate buffer (pH 4.7), the resultant was centrifuged at a rate of 10,000 rpm at 4°C for 5 minutes using a high speed centrifuge. Subsequently, the bacterial pellet was suspended in 20 mM citrate buffer added with 1 M NaCl (pH 4.5; 30 mL), and the suspension was aliquoted into six stainless steel disruption bottles containing glass beads (12 g). The disruption bottles were washed with 20 mM citrate buffer added with 1 M NaCl (pH 4.7; 20 mL) so that each bottle has the total volume of 20 mL. To prevent overheating, the disrupted bottles were stored on ice and the process of disrupting bacteria for 2 minutes and storing on ice for 2 minutes was repeated 7 times. The disrupted bacteria was transferred into fresh 50 mL tubes and centrifuged at a rate of 3,000 rpm at 4°C for 15 minutes using a high speed centrifuge. Then, the supernatant was transferred into a 50 mL conical tube and centrifuged at a rate of 15,000 rpm at 4°C for 10 minutes using a high speed centrifuge. The pellet was suspended in 20 mM citrate buffer (pH 4.7; 10 mL), added into 20 mM citrate buffer (pH 4.7; 10 mL) containing 1% sodium dodecyl sulfate (SDS), and adjusted to a final SDS concentration of 0.5%. The resultant was heat-treated in a constant temperature water bath kept at 60°C for 30 minutes and centrifuged at a rate of 15,000 rpm at 4°C for 5 minutes and the resulting supernatant was removed. After treating with SDS, which is an anion surfactant, the phenomenon that the pellet color turned white was observed. For the removal of SDS, it was suspended in 20 mM citrate buffer (pH 4.7; 20 mL) and centrifuged at a rate of 15,000 rpm at 20°C for 5 minutes using a high speed centrifuge to remove the supernatant. The pellet was washed with 20 mM citrate buffer (pH 4.7; 30 mL) containing 1 M NaCl. For the complete removal of the remaining SDS, the pellet was suspended in injection water, which was heated to 30°C, and centrifuged at a rate of 15,000 rpm at 20°C for 5 minutes. The washing was repeated until no bubble was generated when the pellet was added with the injection water and shaken. The pellet in the state of no bubble was suspended in injection water (10 mL) and stored at room temperature for 10 minutes. The supernatant was transferred to a fresh 50 mL tube while preventing the transfer of the settled glass beads, and centrifuged at a rate of 15,000 rpm at 20°C for 5 minutes to prepare a pellet. For lyophilization, the pellet was suspended with injection water (15 mL), frozen at -80°C, and lyophilized to obtain insoluble WTA-PGN.

### <2-2> Preparation of β-lytic enzyme

For the preparation of β-lytic enzyme, which is used to prepare soluble WTA-PGN from insoluble WTA-PGN, the method in the reference [Li et al., Journal of Biochemistry 122, 772 - 778, 1997] was referred to.

First, crude achromopeptidase (5 g) was dissolved in 10 mM sodium citrate buffer (pH 6.0; 500 mL) and centrifuged at a rate of 15,000 rpm at 4°C for 15 minutes. After equilibrating the supernatant with 10 mM sodium citrate buffer (pH 6.0), the supernatant was eluted by loading it into a CM-Sepharose Fast Flow column (3 cm (length) x 18 cm (width)) followed by a linear gradient performed to a concentration of 0.5 M NaCl. The absorbance of the eluted solution was measured at 280 nm and the fractions showing lytic activity were collected and concentrated. The concentrated samples were subjected to a size exclusion chromatography in a Sephacryl S-100 column (1.6 cm (length) x 87 cm (width)) using a 10 mM sodium citrate buffer (pH 6.0) containing 200 mM NaCl. The absorbance of the filtrate was measured at 280 nm and the fractions showing high lytic activity were collected and concentrated. Among the fractions, the fraction for β-lytic enzyme was selected based on the result of Li *et al.* (1997). Then, β-lytic enzyme was obtained by performing a size exclusion chromatography using a 10 mM sodium citrate buffer (pH 6.0) containing 200 mM NaCl in a Superdex-75 column (1 cm x 30 cm).

The lytic activity or dissolving activity of the thus-obtained β-lytic enzyme was confirmed by culturing *Micrococcus luteus* (ATCC 9341) or a PGN suspension and a column fraction derived from insoluble *Staphylococcus aureus.* As a result of the analysis of the N-terminal sequence by the Procise^{®} Protein sequencer (Cat. No. 491-0, Applied Biosystems, Stafford, TX, USA), the N-terminal sequence was confirmed to be S-P-N-G-L-L-Q-F-P-F, and as a result of the electrophoresis, a single band with a molecular weight of about 25 kDa was observed thus confirming the thus-obtained β-lytic enzyme was β-lytic enzyme.

### <2-3> Purification of soluble WTA-PGN

### ① β-Lytic enzyme treatment and HPLC

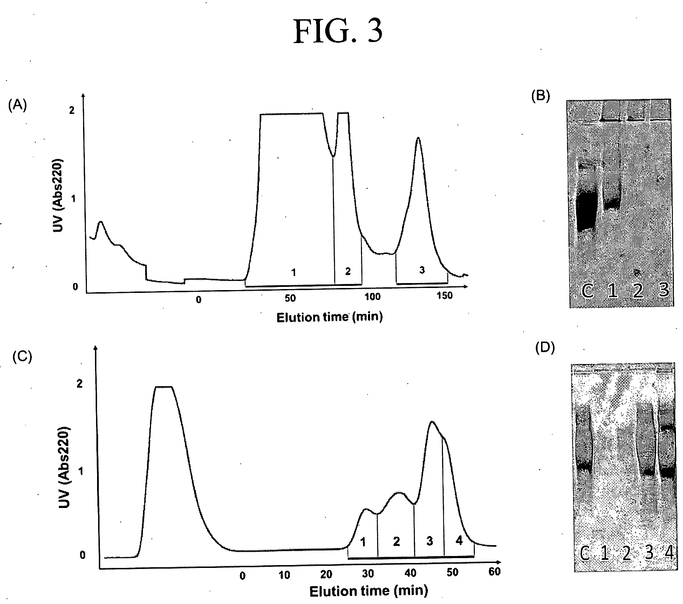

The process of suspending the insoluble WTA-PGN lyophilized in Example <2-1> in 20 mM Tris-HCl (pH 7.0) in a ratio of (100 mg/10 mL) and removing the supernatant by centrifugation was repeated 3 times and confirmed that the supernatant has a pH of 7.0. Then, the insoluble WTA-PGN was added with β-lytic enzyme quantitated by Bradford method, in a ratio of 350 µg of β-lytic enzyme/100 mg WTA-PGN, and reacted them in a 37°C incubator for 12 hours while stirring at a rate of 180 rpm. Then, the reactants were placed in a water bath having a constant temperature of 60°C for 10 minutes to inactivate the enzyme, and centrifuged at a rate of 15,000 rpm at 4°C for 10 minutes to obtain a supernatant. The supernatant was passed through a 0.45 µm filter and the filtrate was loaded into a Hitrap Q column equilibrated with 20 mM Tris-HCl (pH 7.0) and eluted to 20 mM Tris-HCl (pH 7.0) containing 1 M NaCl by linear gradient. As a result of the elution, as shown in FIG. 3A, a total of 3 peaks were obtained. Each of the peaks was named as A, B, and C, and a 27% PAGE was performed (FIG. 3B). Among them, No. 1 fraction stained with silver nitrate on electrophoresis was precipitated with acetone, and lyophilized to obtain soluble WTA-PGN.

### ② lysozyme treatment and HPLC

The lyophilized soluble WTA-PGN (100 mg) was dissolved in 20 mM Tris-HCl (pH 7.0; 10 mL), added with lysozyme (Cat. No. 62970, Sigma-Aldrich Co. LLC., Saint Louis, MO, USA; 1.25 mg), and reacted in a 37°C incubator for 12 hours while stirring at a rate of 180 rpm. Then, the reactants were placed in a water bath having a constant temperature of 60°C for 10 minutes to inactivate the enzyme, and centrifuged at a rate of 15,000 rpm at 4°C for 10 minutes to obtain a supernatant. The supernatant was passed through a 0.45 µm filter and the filtrate was loaded into a Hitrap Q column equilibrated with 20 mM Tris-HCl (pH 7.0) and eluted with 20 mM Tris-HCl (pH 7.0) containing 1 M NaCl by linear gradient. As a result of the elution, as shown in FIG. 3C, a total of 4 peaks were obtained. The peaks were precipitated with acetone and then subjected to electrophoresis (FIG. 3D). Among them, Nos. 3 and 4 fractions being stained with silver nitrate on electrophoresis were collected and lyophilized to obtain soluble WTA-PGN.

### ③ Purification of WTA-PGN using Sephacryl S-200 HR column

The isolated WTA-PGN was further purified by a Sephacryl S-200 HR column. In this step, the HPLC device (805 MANOMETRIC MODULE, 811C DYNAMIC MIXER, 305 PUMP, 306 PUMP, 151 UV/VIS Detector, Gilson, USA) and the Sephacryl S-200 HR, 25 µm to 75 µm (Cat. No. 17-0584-01, GE Healthcare Life Sciences, England) column were used.

The soluble WTA-PGN (53.5mg), isolated previously, was dissolved in distilled water (400 µL) and added into a HPLC injector. Before the elution of the sample, a Sephacryl S-200 HR column was connected to the HPLC injector, washed with distilled water, and was quilibrated, and in order to prevent errors due to the influx of impurities during the experiment, a solvent was flowed thereto at a flow rate of 0.3 mL/min, sensitivity 2, peak width of 10.0 seconds and UV absorbance at 220 nm until the UV detector became stabilized. When UV values are stabilized, the sample was slowly injected by converting the injector into a load mode and upon the initiation of the elution the injector was converted into an injection mode to thereby flowing into a column and obtain the eluate. The process was performed in the same conditions as in the equilibration.

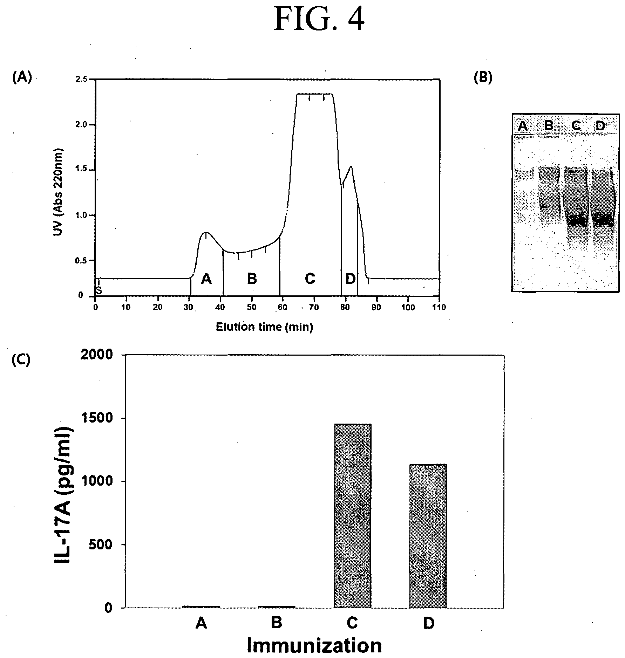

As a result of the elution, as shown in FIG. 4A, a total of 4 peaks (A, B, C, and D) were identified. As a result of PAGE analysis of the peaks, as shown in FIG. 4B, it was confirmed that soluble WTA-PGN was present in peaks B, C, and D.

Each of the thus-obtained 4 peaks was lyophilized and injected into the peritoneum of a mouse. The amount of IL-17A produced as a result was compared and the peak B was shown to have the highest activity (FIG. 4C) and thus B peak was used in the subsequent experiment.

### ④ Purification of WTA-PGN using C18 reverse phase column

The isolated WTA-PGN was further purified by a C18 reverse phase column. The HPLC device (805 MANOMETRIC MODULE, 811C DYNAMIC MIXER, 305 PUMP, 306 PUMP, 151 UV/VIS Detector, Gilson, USA), the Symmetry Shield™ RP18 (5 µm, 4.6 mm x 250 mm) column (Cat. No. 186000112, Waters, Ireland) and the MF™ Membrane filters 0.45 µm (Cat. No. HAWP04700, Merck, Germany) were used. Additionally, Speed Vac (Cat. No. CVE-100, EYELA, Japan), Evaporator (Cat. No. CCA-1110, EYELA, Japan), and a lyophilizer (Cat. No. FDU-2200, EYELA, Japan) were used.

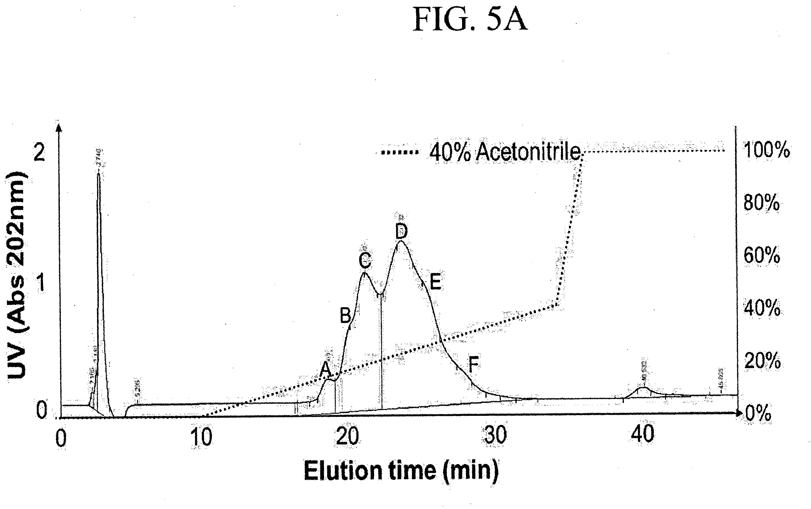

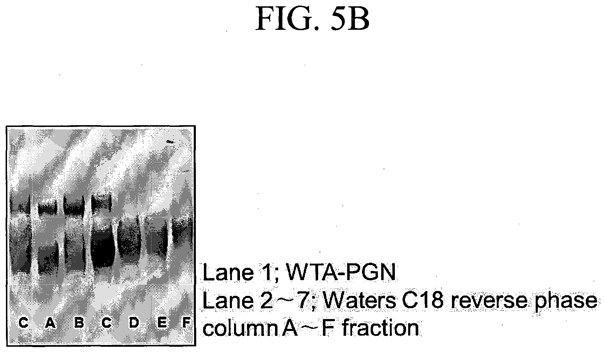

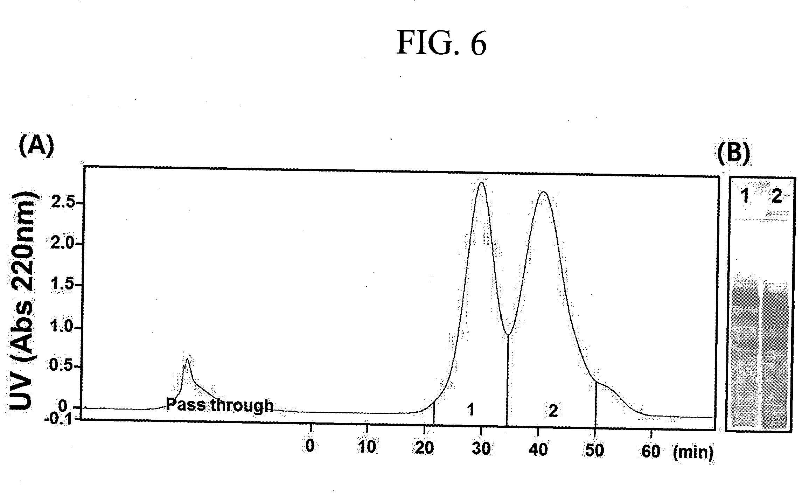

For the purification of WTA-PGN using the C18 reverse phase column, fraction B (see FIG. 4) separated by Sephacryl S-200 column in an amount of 3 mg was dissolved in a stationary phase solvent (200 µL) and used as a sample. The flow rate was 1 mL/min and the UV absorbance was measured at 202 nm. The sample was loaded under the conditions of the sensitivity of 1 and column temperature of 40°C. Elution was performed by setting the concentration gradient of mobile phase at 0% for 10 minutes, at 41.7% for 25 minutes, at 100% for 2 minutes, and at 100% for 10 minutes. As a result of the elution, as shown in FIG. 5A, 6 peaks (A to F) were identified. The peaks were subjected to PAGE analysis and the results are shown in FIG. 5B. The pH of each of the eluted peaks was adjusted to a neutral pH of about pH 6.5 to 7.5 with 1 M Tris-HCl, concentrated to 300 µL using an evaporator, and added with cold acetone (900 µL) and precipitated on ice for one hour. Then, the resultant was centrifuged at 15,000 rpm at 4°C for 25 minutes to recover pellets, and the remaining acetone was dried by speed vac, dissolved in distilled water (20 µL) and then lyophilized.

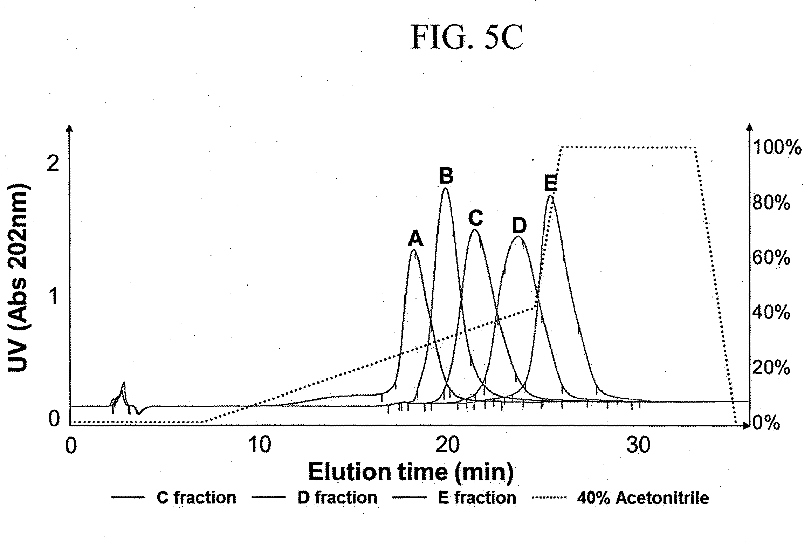

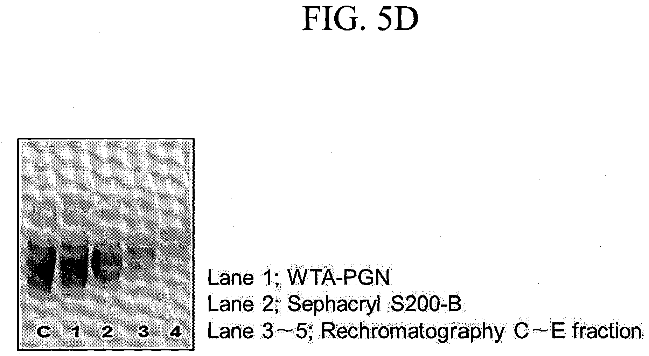

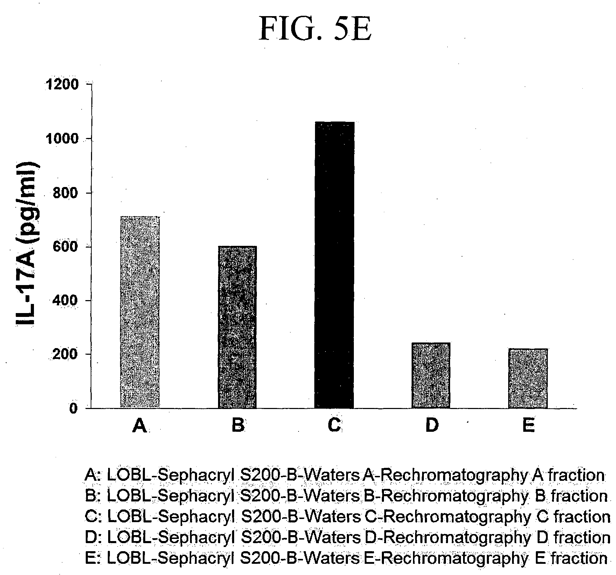

Each fraction (2 mg) was re-separated by loading it again into the C18 column and thereby further purified A, B, C, D, and E fractions were obtained (FIG. 5C). The peaks were subjected to PAGE analysis and the results are shown in FIG. 5D. Additionally, the A, B, C, D, and E fractions, in an amount of 20 µg each, were injected into the peritoneum of mice. The amount of IL-17A induced 6 hours after the injection was quantitated by ELISA and the results are shown in FIG. 5E. As shown in FIG. 5E, fraction C was shown to have the highest amount of IL-17A and thus fraction C was used in the subsequent experiment.

### Example 3: Purification of soluble WTA

For the purification of WTA, insoluble WTA-PGN (80 mg) was suspended in 20 mM citrate buffer (pH 4.5; 19 mL) and added with trichloroacetic acid (100 mg/mL; 1 mL) to a final concentration of 5 mg/mL. The suspension was reacted in a 30°C incubator for 12 hours while stirring at 180 rpm, and then centrifuged at 10,000 rpm at 4°C for 10 minutes. The supernatant was transferred into a 50 mL tube, precipitated with acetone for one hour, and centrifuged at 15,000 rpm at 4°C for 25 minutes. The resulting pellet was transferred into a 1.5 mL microcentrifuge tube to remove acetone and suspended in 20 mM Tris-HCl buffer (pH 7.0; 1 mL).

Then, the resultant was subjected to HPLC using a Hitrap-Q column. All the lines and columns were washed with A buffer, which is 20 mM Tris-HCl (pH 7.0), and the detector was set to detect under a sensitivity of 1 and measure the absorbance at 220 nm, and equilibrated. The sample for loading was filtered with a 0.45 µm filter and loaded. The flow rate was set at 0.5 mL/min so that the sample could bind to the Hitrap-Q column, and the Hitrap-Q column was washed to remove impurities while maintaining the original flow rate until the equilibrium was achieved. A gradient was applied by setting the time required for the buffer B, which consists of 20 mM Tris-HCl and 1 M NaCl (pH 7.0), became from 0 % to 100% as 50 minutes, and upon elution of the WTA attached to the Hitrap-Q column, the sample was recovered from each peak. As a result of the elution, as shown in FIG. 6(A), two peaks (peaks 1 and 2) were obtained. The eluate was aliquoted into 50 mL conical tubes to contain less than 10 mL in each tube and precipitated with cold acetone for 1 hour. During the acetone precipitation, peak 1 showed a yield of 15 mg and peak 2 showed a yield of 0.88 mg. The eluate was centrifuged at 15,000 rpm for 25 minutes to recover pellets, and all acetone was removed by evaporation, and the pellets were dissolved in injection water and lyophilized. The lyophilized product was prepared into a stock (10 mg/mL) and separated by 27% PAGE in an amount of 20 µg each and stained with silver nitrate. As a result, peak 1 is predicted to have a higher number of ribitol and also a higher amount than peak 2 (FIG. 6(B)) and thus peak 1 was used as soluble WTA.

### Example 4: Purification of soluble PGN

### <4-1> TCA treatment for removal of WTA

For obtaining PGN from the lyophilized insoluble WTA-PGN, WTA was removed with trichloroacetic acid (TCA). The insoluble WTA-PGN treated with TCA for obtaining WTA was added and resuspended in 20 mM citrate buffer (pH 4.5; 19 mL), and TCA (100 mg/mL; 1 mL) was added thereto to a final concentration of 5 mg/mL. The resultant was reacted in a 30°C incubator for 12 hours while stirring at 180 rpm, and centrifuged at 10,000 rpm at 4°C for 10 minutes. The resulting pellets were suspended in sterile distilled water (30 mL) and centrifuged at 10,000 rpm at 4°C for 10 minutes, and 5 times and washed to completely remove TCA by repeating the entire process. The resultant was suspended in injection water (15 mL) and lyophilized to obtain insoluble PGN.

### <4-2> β-Lytic enzyme treatment for the purification of insoluble PGN into soluble PGN

The lyophilized insoluble PGN (100 mg) was suspended in 20 mM Tris-HCl (pH 7.0; 10 mL) to a concentration of 10 mg/mL, and 15,000 rpm at 4°C for 10 minutes, and the resulting pellet was washed 3 times with 20 mM Tris-HCl (pH 7.0; 30 mL). The washed insoluble PGN was suspended in 20 mM Tris-HCl (pH 7.0; 20 mL), and 350 µg of purified β-lytic enzyme was added per 100 mg of insoluble PGN and adjusted to have the pH 7.0, and reacted in a 37°C incubator for 12 hours while stirring at 180 rpm. Then, for inactivation, the resultant was heat-treated at 100°C for 10 minutes, cooled at room temperature for at least 10 minutes, and centrifuged at 15,000 rpm at 4°C for 10 minutes. The resulting supernatant was filtered with a 0.45 µm filter and lyophilized.

### <4-3> Purification of soluble PGN by gel filtration chromatography

The lyophilized solubilized PGN was dissolved in injection water to a concentration of 20 mg/mL and then loaded into HiTrap Q, which is an ion exchange column, to remove WTA and WTA-PGN which were contained in a trace amount therein. For the HPLC conditions, equilibrium was adjusted with injection water, which is buffer A, added with 20 mg/mL, and under the flow rate at 1 mL/minute, absorbance at 220 nm, and sensitivity of 1, and a gradient was set with injection water containing 1 M NaCl, which is buffer B, from 0% to 100% for 30 minutes.

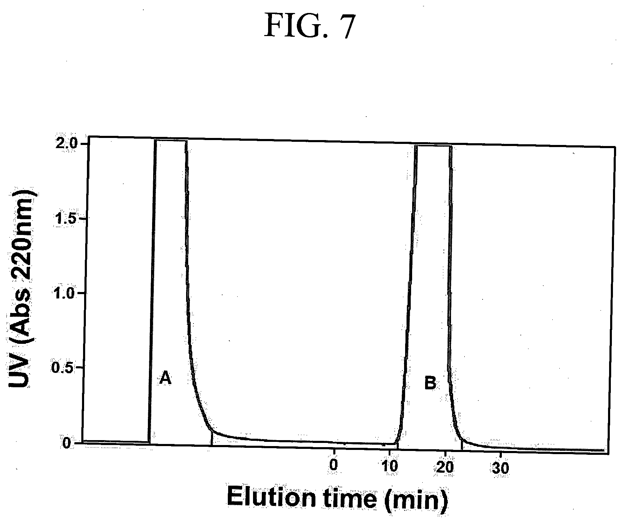

The elution results are shown in FIG. 7. As shown in FIG. 7, the passed solution (fraction A) was assumed to be a fraction containing soluble PGN, in which WTA was removed, and the fraction (fraction B), which is eluted by buffer B containing 1 M NaCl, was assumed to be WTA-PGN or WTA, in which WTA was not removed. Accordingly, only fraction A was collected and lyophilized.

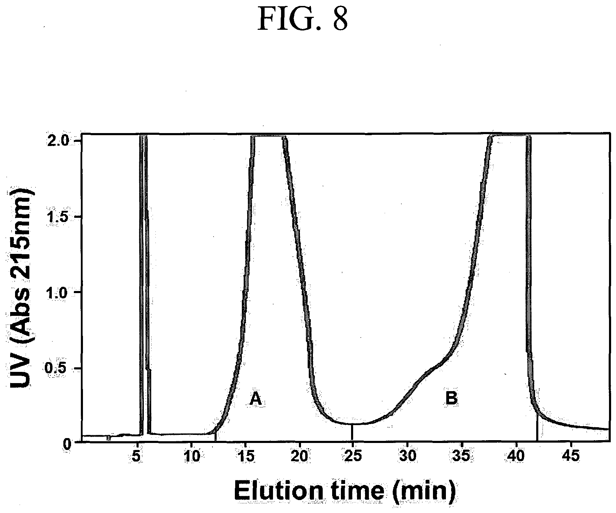

Then, for the purification of soluble PGN with a predetermined length of glycan, the lyophilized product was dissolved in injection water at a concentration of 20 mg/mL and loaded into a Toyopearl HW 55 S (Cat. No. 14686, TOSOH Bioscience, Japan) column to perform gel filtration. With respect to the gel filtration conditions, the equilibrium was adjusted with injection water and proceeded at a flow rate of 0.5 mL/minute, absorbance of 215 nm, sensitivity of 1, and operation for 50 minutes. The elution pattern by the gel filtration is shown in FIG. 8. As shown in FIG. 8, two peaks were confirmed during gel filtration. Between the peaks, in order to confirm that peak B is soluble PGN, the pro-phenoloxidase system of *Tenebrio molitor,* an insect, was used. The peak was continuously diluted up to a 10⁶-fold. β-1,3 was used as a positive control, and when compared considering the OD value of the negative control as the baseline, it was confirmed that activities were shown both in peaks A and B (FIG. 8). Since low molecular weight mateirals come later due to the characteristics of the Toyopearl HW 55 S column, it can be confirmed that peak B is a soluble PGN having a lower molecular weight than peak A, *i.e.,* with a small number of saccharides. Peak B was separated and lyophilized and used for subsequent experiments.

### Example 4: Validation of biochemical characteristics of WTA-PGN and WTA

For the validation of biochemical characteristics of isolated/purified WTA-PGN and WTA, 27% PAGE and silver nitrate staining were performed. The presence *of D-*Ala was analyzed by silica gel thin layer chromatography, and the amount of phosphate and GlcNAc residue present in WTA was quantitated by a known analysis method.

Specifically, to confirm the presence of D-Ala, a sample for identification (10 mg/mL) in an amount of 2 µL and 1 M NaOH (0.2 µL) were added into a microcentrifuge tube and cultured in a 37°C incubator for 2 hours while stirring at 180 rpm. The thin-layer chromatography film was cut into a size of 5 cm (length) x 10 cm (width), and the sample (2 µL) was loaded about 2 cm from the top not to directly touch the TLC solution and allowed to develop for 1 hour and 30 minutes. Then, the thin-layer chromatography film was completely dried, sprayed with ninhydrin spray reagent (1 mL), and heat-treated in the heatblock until a violet-red spot appeared.

Additionally, for the quantitation of phosphate, a sample (2 µL to 4 µL), distilled water (100 µL), and a digestion reagent (175 µL) were added into a glass tube and vortexed. After heating the tube from the outside and confirming that the sample color was turned to thick yellow, the tube was heated again until the color became thin. The resultant was cooled at room temperature for 10 minutes, added with distilled water (125 µL) and 1% ammonium molybdate (1 mL), and stirred. Then, a reducing agent (50 µL) was added thereto, heated in a constant temperature water bath for 10 minutes, and the OD values were measured at 750 nm using a spectrophotometer. As the standard material, phosphate was used.

Additionally, for the quantitation of GlcNAc, the sample separated by microcentrifugation, distilled water, and 6.45 N HCl were added to a final volume of 100 µL, vortexed, and transferred into a small glass tube (0.5 cm (diameter) x 5 cm (height)), and all the bubbles contained in the sample was removed using a vacuum pump device. The resultant was heat-treated in a 100°C oven for 3 hours and cooled at room temperature. Then, the resultant was transferred into a large glass tube (diameter 1.8 cm x height 18 cm) using a Pasteur pipette and heated while preparing a vacuum state by placing it under vacuum thereby drying the sample. Subsequently, a solution (ethanol : distilled water : trimethylamine = 2 : 2 : 1) in an amount of 50 µL was added to each tube and dried two times. As such, all HCl contained in the sample was removed, added with distilled water (100 µL), anhydrous acetic acid (20 µL), and borate buffer (100 µL) and mixed, and boiled in a 95°C constant temperature water bath for 8 minutes, and cooled at room temperature. *p*-Dimethylaminobenzaldehyde reagent (750 µL) and 2-ethoxy ethanol (50 µL) were added thereto, cultured in a 20°C constant temperature water bath for 15 minutes, and the OD values were measured at 585 nm using a spectrophotometer. As a standard material, *N*-acetylglucosamine was used.

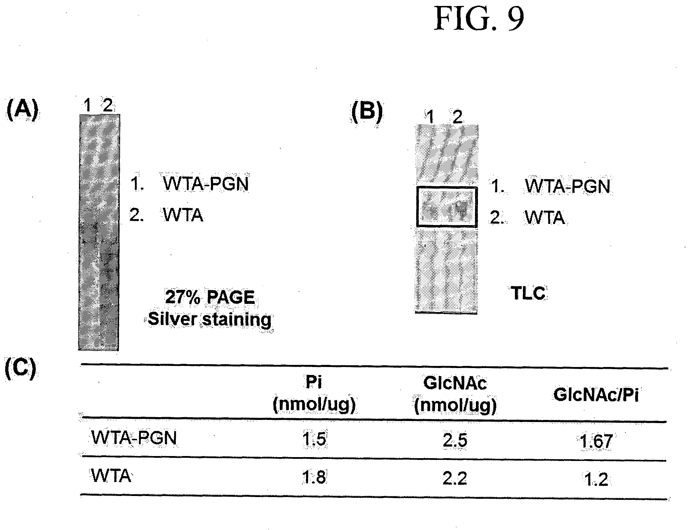

As a result, as shown in FIG. 9A, the gel mobility of the purified WTA was shown to be faster than the purified WTA-PGN, and this confirms that WTA, by removing PGN, has a smaller molecular weight than WTA-PGN. Additionally, as shown in FIG. 9B, the D-Ala residue was shown to be bound to both WTA-PGN and WTA. Additionally, as shown in FIG. 9C, the phosphate content was shown to be 1.5 nmol/µg in WTA-PGN, 1.8 nmol/µg in WTA, and the GlcNAc content was shown to be 2.5 nmol/µg in WTA-PGN and 2.2 nmol/µg in WTA.

### Example 5: Experimental method for analyzing the effects of WTA-PGN

### <5-1> Design of mouse experiments and breeding

As experimental animals, 5-week-old specific pathogen-free (SPF) C57BL/6J female mice (body weight; 15 ± 0.5 g) were obtained from the Biomedical Mouse Resource Center, the Korea Research Institute of Bioscience and Biotechnology (KRIBB) (Ohchang Campus, Chungcheongbuk-do, Korea, a *rea* gene defect). The experimental animals were adapted to the animal experiment laboratory environment (a cage for animals with constant-temperature constant-humidity, Cat. No. AAAC2051, JEIO TECH Co., Ltd., a *rea* gene defect; at 20°C to 25°C with 55% humidity) for a week while feeding them with commercial solid feeds (Cat. No. 5L79, Orient Bio Inc., a *rea* gene defect), before the experiment. The mice were divided into each group (6 to 12 mice/group) by a completely randomized design according to the body weight and placed them into breeding cages (6 mice/group) and *ad libitum* fed with diets and drinking water. The body weight and dietary intake of each experimental animal were measured once daily, and the illumination was turned on and off at 12-hour intervals.

### <5-2> Preparation of MRSA (S. aureus USA300 strain) and MSSA (S. aureus NRS184 strain) to be used for the study of in-vivo infection

MRSA (a S. *aureus USA300* strain) and MSSA (a S. *aureus* NRS184 strain) were provided from Tubingen university of Germany. The strains stored at -80°C in glycerol stocks were plated on LB Broth LENNOX (Cat. No. LBL405.1, Bioshop, Canada) and cultured in a 37°C incubator for at least 12 hours. Then, the cultured plates were stored at 4°C. One day before the use of the strains, Bacto™ Tryptic Soy Broth Soybean-Casein Digest Medium (Cat. No. 211825, BD, USA New Jersey; 2 mL) was added into a 14 mL round-bottom tube and inoculated with one colony and cultured in a 37°C incubator while stirring for at least 12 hours. Subsequently, 3 hours before infecting the animals, the bacterial culture broth (400 µL) cultured at 37°C for 12 hours was added into TSB (20 mL) and cultured in a 37°C incubator while stirring to develop into a mid-log phase. The bacterial culture broth, upon completion of the cultivation, was stored to prevent cell proliferation and allowed to progress further. First, the resultant was centrifuged at a rate of 3,000 rpm at 4°C for 10 minutes, and the supernatant was removed. The resultant was suspended in PBS and centrifuged at a rate of 15,000 rpm at 4°C for 1 minute. After centrifugation, the supernatant was removed, resuspended in PBS (1 mL), and the absorbance was measured at 600 nm using a spectrophotometer (Cat. No. 206-25400-58, Shimadzu, Japan). The suspension was diluted considering the necessary number of cells, according to each animal model and infection method, and used in the experiments.

### <5-3> Preparation of heat-killed bacteria

The amount of IL-17A was measured using the heat-killed *S. aueus* strains shown in Table 1.

| **Strain** | **Genotype** | **Phenotype** | **Ref.** |
|---|---|---|---|
| *RN4220* | *Patent strain* | Parent strain | (1) |
| *USA300* | *USA300* | MRSA strain | (2) |
| *MW2* | *MW2* | MSSA strain | (3) |
| *T002* | *ΔoatA::Erm* | *O*-acetyltransferase depleted | (4) |
| *T013* | *Δlgt::Erm* | Lipoprotein lipidation depleted | (5) |
| *T174* | *ΔtagO::Erm* | WTA depleted | (6) |
| *M0793* | *ΔdltA::Erm* | *D*-alanine modification of WTA and LTA | (6) |
| *T384* | *ΔoatA,Δlgt::PheO,Erm* | *O*-acetyltransferase and Lipoprotein depleted | (7) |
| *T895* | *ΔoatA,Δlgt,ΔdltA::PhleO,Erm* | *O*-acetyltransferase, Lipoprotein and D-alanine depleted | (7) |
| *T899* | *ΔoatA*/*ΔlgtΔtarM::PhleO,Erm* | *O*-acetyltransferase, Llipoprotein and β-GlcNAc of WTA depleted | (7) |
| *T901* | *ΔoatA*/*Δlgt*/*ΔtarS::PhleO,Km* | *O*-acetyltransferase, Lipoprotein and α-GlcNAc of WTA depleted | (7) |
| *T878* | *ΔoatA,Δlgt,ΔtarM,ΔtarS::Phle O,Erm,Km* | *O*-acetyltransferase, Lipoprotein and α,β-GlcNAc of WTA depleted | (7) |

| | | | |
|---|---|---|---|
| (1) Novick RP, Ross HF, Projan SJ, Kornblum J, Kreiswirth B, Moghazeh S. (1993) Synthesis of Staphylococcal Virulence Factors Is Controlled by a Regulatory Rna Molecule. EMBO J. 12 (10): 3967- 75. (2) From Cellular and Molecular Microbiology Division, Interfaculty Institute of Microbiology and Infection Medicine, University of Tübingen, Germany (3) Kurokawa K, Kim MS, Ichikawa R, Ryu KH, Dohmae N, Nakayama H, Lee BL. (2012) Environment-mediated accumulation of diacyl lipoproteins over their triacyl counterparts in Staphylococcus aureus. J Bacteriol. 194 (13): 3299 - 306. (4) Park KH, Kurokawa K, Zheng L, Jung DJ, Tateishi K, Jin JO, Ha NC, Kang HJ, Matsushita M, Kwak JY, Takahashi K, Lee BL. (2010) Human serum mannose-binding lectin senses wall teichoic acid Glycopolymer of Staphylococcus aureus, which is restricted in infancy. JBiol Chem. 285 (35): 27167 - 75. (5) Nakayama M, Kurokawa K, Nakamura K, Lee BL, Sekimizu K, Kubagawa H, Hiramatsu K, Yagita H, Okumura K, Takai T, Underhill DM, Aderem A, Ogasawara K. (2012) Inhibitory receptor paired Ig-like receptor B is exploited by Staphylococcus aureus for virulence. J Immunol. 189 (12): 5903 - 11. (6) Kaito C, Sekimizu K. (2007) Colony spreading in Staphylococcus aureus. J Bacteriol. 189 (6): 2553 - 7. (7) Takahashi K, Kurokawa K, Moyo P, Jung DJ, An JH, Chigweshe L, Paul E, Lee BL. (2013) PLoS One. 8 (8): e69739. | | | |

LB10 broth medium (2 mL) was added with antibiotics using the strains described in Table 1 above. With respect to genotypes, Erm represents erythromycin (Cat. No. E6376, Sigma-Aldrich Co. LLC., Saint Louis, MO, USA), Km represents kanamycin (Cat. No. 17924, USB^{®}, OH, USA), and they were used at concentrations of 10 µg/mL, and 50 µg/mL, respectively. PhleO represents phleomycin (Cat. No. P9564, Sigma-Aldrich Co. LLC., Saint Louis, MO, USA) and is used at a concentration of 10 µg/mL for classification and confirmation of each mutant type during the preparation of strain, but it was not used for culturing bacteria. Colonies of each strain were inoculated and cultured for 12 hours, and 1 mL of the bacterial culture broth of each strain, which was subcultured by preparing LB10 (100 mL) in a 500 mL Erlenmeyer flask, was added thereto, and cultured according to the growth temperature while stirring at a rate of 180 rpm.

The bacteria were cultured until the OD₆₀₀ₙₘ reached 1.2, and the resulting bacteria culture was centrifuged at a rate of 10,000 rpm at 4°C for 10 minutes. The resulting bacteria pellet was suspended in physiological saline (0.9% NaCl; 30 mL) and centrifuged at a rate of 10,000 rpm at 4°C for 10 minutes and the supernatant was removed. After washing twice with physiological saline, the pellet was resuspended in saline (10 mL). The suspension was diluted in 50 mL tubes to adjust the OD₆₀₀ₙₘ to 0.3 and added into a 60°C constant temperature water bath, and heat-treated for 30 minutes. The process of sufficiently cooling the heat-treated bacteria at room temperature, centrifuging at a rate of 10,000 rpm at 4°C for 10 minutes, removing the supernatant and suspending the pellet in injection water (30 mL) was repeated and washed twice with injection water. The pellet was suspended in injection water (2 mL) and the weight after lyophilization was measured. Then, the resultant was intraperitoneally injected into mice at a concentration of 200 µg/mL.

### <5-4> Cellular immunity induced by WTA-PGN in a mouse model

### (i) Peritonitis, cellular immunity, and immune response induced by WTA, PGN, and WTA-PGN

Heat-killed bacteria or a WTA derivative in an amount of 50 µg, 100 µg, and 200 µg was intraperitoneally was suspended in 100 µL PBS (Cat. No. 17-516Q, BioWhittaker^{®}, LONZA, MD, USA) and intraperitoneally injected into mice thereby inducing peritonitis or immunizing the mice, and the mice were sacrificed, at sampling time-points of 0-, 3-, 6-, 9-, 12-, 24-, 48-, and 72 hours, necessary for each experiment and used in the experiments. The experimental model for repeated induction of peritonitis and memory response was intraperitoneally injected on day 0, day 7, and day 14 with 100 µg of WTA, PGN, and WTA-PGN in 100 µL of PBS, respectively, allowed to go through with a recovery period for 21 days, and infected with intraperitoneal injection of MRSA (*S. aureus USA300* strain, 1 x 10⁸ CFU/100 µL PBS) on day 35. The mice in the control group were intraperitoneally injected with 100 µL of PBS and naive mice were used as a control. After the bacterial challenge, the mice were sacrificed at time-points of 0-, 3-, 6-, 9-, 12-, 24-, 48-, and 72 hours, and the systemic infection level and immune responses were evaluated. Meanwhile, the groups which survived MRSA (*S. aureus USA300* strain) and MSSA (*S. aureus* NRS184 strain) were intraperitoneally injected with 5 x 10⁸ CFU in 100 µL PBS.

### (ii) Separation of peritoneal fluid exudate and peritoneal exdudate cells (PEC)

With respect to the mouse peritoneal fluid exudate, the peritoneum was washed with 2 mL of PBS (Cat. No. 17-516Q, BioWhittaker^{®}, LONZA, USA), centrifuged at 2,000 rpm for 10 minutes. The supernatant was stored at -80°C and used for the analysis of cytokines by ELISA and the pellet was resuspended in complete RPMI 1640 medium (cRPMI; RPMI 1640: Gibco^{®}; 10% FBS: Gibco^{®}; 100 mM L-glutamine: Gibco^{®}; and 100 mg/mL penicillin/streptomycin: Gibco^{®}). For the removal of red blood cells, the red blood cells were lysed using a red blood cell lysis buffer (Cat. No. 420301, Bio legend, San Diego, CA, USA) and the cells were resuspended again in cRPMI.

### (iii) Cytokine analysis by ELISA

With respect to IL-17A, IL-23, IL-1β, IL-10, and IFNγ, they were measured in the supernatant of the peritoneal fluid exudate using the Duoset^{®} ELISA kit (R&D Systems Inc., Minneapolis, MN, USA) of R & D and ELISA Ready-SET-Go! Kit (eBioscience, San Diego, CA, USA) of eBioscience. The ELISA kit information on each cytokine is shown below. Finally, the absorbance value measured at 450 nm was amended to the absorbance value measured at 550 nm using a microplate reader (Cat. No. 51119000, Thermo Fisher Scientific Inc., Waltham, MA, USA).

### (iv) Flow cytometry (FACS)

For staining of the surface, cells were washed with PBS and stained using the flow cytometry Ab. Additionally, for intracellular staining, the cells were re-stimulated in the presence of cRPMI1640 medium with Golgistop (Cat. No. 554724, BD Bioscience, San Jose, CA, USA), which is a protein transport inhibitor, before collecting the cells. The collected cells were washed with PBS, fixed at room temperature with 100 µL of an intracellular (IC) immobilization buffer (Cat. No. 00-8222-49, eBioscience, San Diego, CA, USA) for 30 minutes and then washed with a permeabilization buffer (Cat. No. 00-8333-56, ebioscience, San Diego, CA, USA). Then, the cells were resuspended with 100 µLof a permeabilization buffer and cultured at room temperature for 20 minutes using intracellular staining Ab. Then, cells were washed with a permeabilization buffer and PBS per each step, and resuspended with PBS. The cells were detected by flow cytometry (BD FACS Canto II, BD Biosciences, San Jose, CA, USA) and analyzed using the FlowJo software vX 0.7 (FlowJo LLC., Ashland, OR, USA). The specific information on each surface molecule and intracellular molecule are shown in Table 2 below.

### (v) Cell sorting and in-vitro co-cultivation of macrophages which immunized WTA derivatives, dendritic cells, and purified γδ T cells

The PEC of a naive mouse and a mouse, in which the WTA derivatives are immunized, were isolated as described above, and the PEC was transferred into a 96-well flat bottom plate (2- to 3 x 10⁵ cells/well) and cultured in cRPMI medium at 37°C, 5% CO₂ condition (Cat. No. MCO-17A, SANYO, Japan) for 1.5 hours so that the macrophages and dendritic cells could be attached thereto. Then, the medium was removed by suction and replaced with an RPMI without antibiotics.

The FACS sorting was performed: by negative selection of CD3⁺ T cells using murine Pan T cell sorting kit II (Cat. No. 130-095-130, Miltenyi Biotec, Bergisch Gladbach, Germany) for macrophages and dendritic cells; and using γδ TCR-specific Ab for γδ T cells.

The stained cells were placed under pressure through a round bottom tube with a cell strainer snap cap (Cat. No. 352235, Tewksbury, MA, USA) and sorted using the flow cell sorter (MoFlo® Astrios™ cell sorter, Beckman Coulter, Inc., South Kraemer Boulevard Brea, CA, USA). The purity of the sorted cells was 95% or higher.

### (vi) RNA isolation, cDNA synthesis, and quantitative real-time PCR (qRT-PCR)

The total RNA was isolated using the TRI Reagent^{®} (Cat. No. TR 118, Molecular Research Center, Inc., Cincinnati, OH, USA). RNA isolation was performed according to the manufacturer's protocol, and for cDNA synthesis, mRNA was reversely transcribed to cDNA in a total volume of 20 µL including oligo (dT) primer (Cat. No. C1101, Promega Corporation, Madison, WI, USA) and the Improm-II system (Cat. No. A3800, Promega Corporation, Madison, WI, USA) using the RT-PCR device (C1000 Touch™ Thermal Cycler, Bio-Rad, Hercules, CA, USA). The transcribed cDNA was amplified using the qRT-PCR device (Cat. No. 9001870, Rotor-Gene Q, QIAGEN Inc., Valencia, CA, USA) along with RT-PCR. The data was normalized with hypoxanthine-guanine phosphoribosyltransferase (HPRT) for each condition. The primer information on each gene is shown in Table 3 below.

**[Table 2] Information on FACS antibodies**

| **Molecule** | **Fluorescence** | **Manufacturer** | **Clone** |
|---|---|---|---|
| CD4 | FITC, PE, APC, PercpCy5.5 Brilliant Violet510, PE/Cy7, Brilliant Violet 421 | Biolegend | RM4- |
| TCR γ/δ | Brilliant Violet 421 | Biolegend | GL3 |
| V γ 1.1 | PE | Biolegend | 2.11 |
| V γ 2 | APC | Biolegend | UC3-10A6 |
| Ly-6G (Gr-1) | APC | TONBO | RB6-8C5 |
| CD121a (IL-1Ra) | PE | Biolegend | JAMA-147 |
| CD11b | PerCP/Cy5.5 | TONBO | M1/70 |
| CD44 | PE-Cy7 | TONBO | IM7 |
| CD27 | PE | Biolegend | LG.3A10 |
| CD3 | FITC | TONBO | 17A2 |
| CD8α | PerCP/Cy5.5 | TONBO | 53-6.7 |
| CD11c | Brilliant Violet 421 | Biolegend | N418 |
| MHCII | FITC | TONBO | M5/114.15.2 |
| F4/80 | PE | Biolegend | BM8 |
| CD40 | APC | Biolegend | 3/23 |
| CD80 | PE | Biolegend | 16-10A1 |
| CD86 | APC | Biolegend | GL-1 |
| IFN-γ | PE, PE/Cy7 | eBioscience, Biolegend | XMG1.2 |
| IL-17A | APC, PE/Cy7 | Biolegend | TC11-18H10.1 |

**[Table 3] Information on primers**

| **Gene** | **Primer Sequence** |
|---|---|
| *HPRT* | F: 5'-TTA TGG ACA GGA CTG AAA GAC-3' (SEQ ID NO: 1) |
| | R: 5'-GCT TTA ATG TAA TCC AGC AGG T-3' (SEQ ID NO: 2) |
| *IFN-γ* | F: 5'-GAG CCA GAT TAT CTC TTT CTA CC-3' (SEQ ID NO: 3) |
| | R: 5'-GTT GTT GAC CTC AAA CTT GG-3' (SEQ ID NO: 4) |
| *IL-10* | F: 5'-ATA ACT GCA CCC ACT TCC CA-3' (SEQ ID NO: 5) |
| | R: 5'-TCA TTT CCG ATA AGG CTT GG-3' (SEQ ID NO: 6) |
| *IL-17A* | F: 5'-TTT AAC TCC CTT GGC GCA AAA-3' (SEQ ID NO: 7) |
| | R: 5'-CTT TCC CTC CGC ATT GAC AC-3' (SEQ ID NO: 8) |
| *IL-1β* | F: 5'-CAA CCA ACA GAT ATT CTC C-3' (SEQ ID NO: 9) |
| | R: 5'-TGC CGT CTT TCA TTA CAC AG-3' (SEQ ID NO: 10) |
| *IL-12p40* | F: 5'-GGA ACG ACG GCA GCA GAA TA-3' (SEQ ID NO: 11) |
| | R: 5'-AAC TTG AGG GAG AAG TAG GAA TGG-3' (SEQ ID NO: 12) |
| *IL-23 p19* | F: 5'-TGG CAT CGA GAA ACT GTG AGA-3' (SEQ ID NO: 13) |
| | R: 5'-TCA GTT CGT ATT GGT AGT CCT GTT A-3' (SEQ ID NO: 14) |
| *NLRP3* | F: 5'-AGC CTT CCA GGA TCC TCT TC-3' (SEQ ID NO: 15) |
| | R: 5'-CTT GGG CAG CAG TTT CTT TC-3' (SEQ ID NO: 16) |
| *TLR1* | F: 5'-CCC TAC AGA AAC GTC CTA TAC C-3' (SEQ ID NO: 17) |
| | R: 5'-ATG ATA AGC TCA CAT TCC TCA G-3' (SEQ ID NO: 18) |
| *TLR2* | F: 5'-GAC AAA GCG TCA AAT CTC AG-3' (SEQ ID NO: 19) |
| | R: 5'-CCA GAA GCA TCA CAT GAC AG-3' (SEQ ID NO: 20) |
| *TLR3* | F: 5'-TAA AGC GAG TTT CAC TTT CAG G-3' (SEQ ID NO: 21) |
| | R: 5'-GCA GTT TAA CTT CCC AGA TAG AG-3' (SEQ ID NO: 22) |
| *TLR4* | F: 5'-CCC TGC ATA GAG GTA GTT CC-3' (SEQ ID NO: 23) |
| | R: 5'-GTT TGA GAG GTG GTG TAA GC-3' (SEQ ID NO: 24) |
| *TLR5* | F: 5'-CAG GAT GTT GGC TGG TTT CT-3' (SEQ ID NO: 25) |
| | R: 5'-CGG ATA AAG CGT GGA GAG TT-3' (SEQ ID NO: 26) |
| *TLR6* | F: 5'-TGC TGG AAA TAG AGC TTG GA-3' (SEQ ID NO: 27) |
| | R: 5'-GGA CAT GAG TAA GGT TCC TG-3' (SEQ ID NO: 28) |
| *TLR7* | F: 5'-CAA GAA AGA TGT CCT TGG CTC-3' (SEQ ID NO: 29) |
| | R: 5'-CCA TCG AAA CCC AAA GAC TC-3' (SEQ ID NO: 30) |
| *TLR8* | F: 5'-TTG CCA AAG TCT GCT CTC TG-3' (SEQ ID NO: 31) |
| | R: 5'-CAT TTG GGT GCT GTT GTT TG-3' (SEQ ID NO: 32) |
| *TLR9* | F: 5'-CCC AAC ATG GTT CTC CGT C-3' (SEQ ID NO: 33) |
| | R: 5'-GGG TAC AGA CTT CAG GAA CAG-3' (SEQ ID NO: 34) |

### <5-5> Cellular immunity induced by WTA-PGN in NZW rabbits and guinea pigs models

### (i) Measurement of protective effects against MRSA infection by WTA-PGN immunization in the skin of NZW rabbits

As experimental animals, NZW female rabbits (Yac; NZW (KBL)) with a body weight of 2 ± 0.1 kg were purchased from Orient Bio Inc. (Gyeonggi-do, Korea), and allowed them to adapt to the animal experiment laboratory environment (a cage for rabbits, Cat. No. DJ117, Daejong Instrument Industry Co., Ltd., Korea; at 20°C to 25°C with 55% humidity) for a week while feeding them with commercial solid feeds (Cat. No. 38302-NM, Cargill Agri Purina, Inc., a *rea* gene defect), before the experiment. Experimental animals were placed into breeding cages one per each cage and *ad libitum* fed with diets and drinking water. The body weight and dietary intake of each experimental animal were measured once daily, and the illumination was turned on and off at 12-hour intervals.

Before the WTA-PGN immunization and infection with MRSA *USA300*) into the skin of NZW rabbits, the hairs on the back of the NZW rabbits with a size of 15 cm (width) x 10 cm (length) were removed using an electric shaver (Cat. No. ER806, Panasonic Corporation, Japan), and the shaved area was disinfected with sterile alcohol cottons and a povidone-iodine solution (Cat. No. P698900, TRC, CANADA) and used in the experiment.

For the anesthetization of animals, Zoletil® 50 (Virbac Korea Co., Ltd., Korea) and Xylazine hydrochloride (Cat. No. 1251, Sigma-Aldrich Co. LLC., Saint Louis, MO, USA) were mixed at a concentration of 30 mg/0.6 mL/2 kg and 9.328 mg/0.4 mL/2 kg, respectively, considering the body weight of animals, and intramuscularly injected.

The shaved area was divided into halves (7.5 cm (length) x 10 cm (width)) and partitioned. The left side as the control was immunized with PBS (100 µL) by intradermal injection and the right side was immunized with WTA-PGN (20 µg/100 µL). One of the rabbits was infected by intradermal injection with *USA300* (1 x 10⁸ CFU) after 3 hours and another rabbit was infected with *USA300* (1 x 10⁸ CFU) by intradermal injection after 6 hours, and the size of the skin abscess lesions was measured by width (w) and length (1), and the dermonecrosis area (cm²) for 7 days and abscess volume (cm³) were quantitated. The abscess volume (cm³) was calculated by the equation [v = (π/6) x 1 x w²] for the spherical ellipsoid.

### (ii) Measurement of protective effect against MRSA infection by WTA-PGN immunization in the skin of guinea pigs

As experimental animals, female guinea pigs (CrlOri; HA) with a body weight of 2 ± 0.1 kg were purchased from Orient Bio Inc. (Gyeonggi-do, Korea), and allowed them to adapt to the animal experiment laboratory environment (a cage for constant-temperature constant-humidity animals, Cat. No. AAAC2051, JEIO TECH Co., Ltd., Korea; at 20°C to 25°C with 55% humidity) for a week while feeding them with commercial solid feeds (Cat. No. 5026, Orient Bio Inc., Korea), before the experiment. Experimental animals were placed into breeding cages one per each cage and *ad libitum* fed with diets and drinking water. The body weight and dietary intake of each experimental animal were measured once daily, and the illumination was turned on and off at 12-hour intervals.

Before the WTA-PGN immunization and infection with MRSA *USA300*) into the skin of guinea pigs, the hairs on the back of the guinea pigs with a size of 15 cm (width) x 10 cm (length) were removed using an electric shaver (Cat. No. ER806, Panasonic Corporation, Japan), and the shaved area was disinfected with sterile alcohol cottons and a povidone-iodine solution (Cat. No. P698900, TRC, CANADA) and used in the experiment.

For the anesthetization of animals, Zoletil^{®} 50 (Virbac Korea Co., Ltd., Korea) and Xylazine hydrochloride (Cat. No. 1251, Sigma-Aldrich Co. LLC., Saint Louis, MO, USA) were mixed at a concentration of 3 mg/0.06 mL/100 g and 0.9328 mg/0.04 mL/100 g, respectively, considering the body weight of animals, and intramuscularly injected.

The shaved area was divided into halves (3 cm (length) x 6 cm (width)) and partitioned. The left side as the control was immunized with PBS (100 µL) by intradermal injection and the right side was immunized with WTA-PGN (20 µg/100 µL). After 6 hours, the size of the skin abscess lesions was infected with *USA300* (5 x 10⁸ CFU) by intradermal injection and measured by width (w) and length (1), and the dermonecrosis area (cm²) for 7 days and abscess volume (cm³) were quantitated. The abscess volume (cm³) was calculated by the equation [v = (π/6) x 1 x w²] for the spherical ellipsoid.

### (iii) Measurement of protective effect against MRSA infection by WTA-PGN immunization in the peritoneum of guinea pigs

The breeding of experimental animals was performed in the same manner as in II.5.2). Using the guinea pigs, the control group (n = 2) was immunized by intraperitoneal injection with PBS (100 µL) and the WTA-PGN group (n = 2) was immunized by intraperitoneal injection with WTA-PGN (200 µg/100 µL). After 3 hours, the guinea pigs were infected with *USA300* (1.5 x 10⁹ CFU) by intraperitoneal injection and the body weight, the amount of dietary intake, the movement, etc., were monitored for 7 days. Then, the guineas pigs were dissected and the presence and size of abscess, the conditions of organs, etc., were observed.

### <5-6> Humoral immunity induced by WTA-PGN

### (i) Immunization into the skin by WTA, PGN, and WTA-PGN derivatives and MRSA (USA300) infection

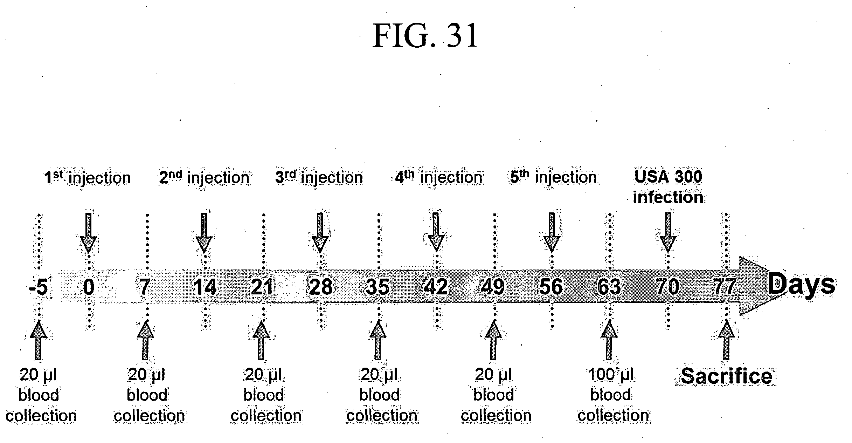

Experimental animals were immunized a total of 5 times by intradermal injection of PBS (Cat. No. 17-516Q, Lonza Walkersville, Inc., MD, USA) and 20 µg of WTA and PGN derivatives in an amount of 50 µL, respectively, on day 0, day 14, day 28, day 42, and day 56. Before the immunization and on day 7, day 21, day 35, day 49, and day 63, *i.e.,* 7 days after the respective immunization, 20 µL of blood samples were collected by cutting the tails. On day 70, which was 14 days after the 5^{th} immunization, the animals were infected with *USA300* (1 x 10⁷ CFU/100 µL of PBS) by intravenous injection, and on day 77, which was 7 days thereafter, the animals were sacrificed. The time schedule used in the experiment was schematized and shown in FIG. 31. The mice were fasted 12 hours before the sacrifice and then anesthetized using an anesthetic (1 µL/g BW), which was prepared by adding 100 mg of 2,2,2-tribromoethanol (Cat. No. T48402, Sigma-Aldrich Co. LLC., Saint Louis, MO, USA) and 200 µL of t-amyl alcohol (Cat. No. 152463, Sigma-Aldrich Co. LLC., Saint Louis, MO, USA) in 0.9% NaCl solution (Cat. No. S 3014, Sigma-Aldrich Co. LLC., Saint Louis, MO, USA). Then, blood samples were collected from the hearts using heparin-treated sterile syringes and centrifuged at a rate of 8,000 rpm at 10°C for 10 minutes and the thus-obtained plasma was used as a sample. The tissues from the liver, spleen, kidney, heart, lung, etc., were ablated and their weights were measured. The kidney tissue was used for CFU measurement and the remaining tissues were dipped into liquid nitrogen and stored at -80°C for later use as samples.

### (ii) Quantitation of antigen-specific antibodies in serum immunized with WTA derivatives by ELISA

Each well of a 96-well microplate was treated with 5 nmol WTA-PGN (50 µL) and coated at 4°C overnight, using goat anti-mouse IgG-FC (Cat. No. G-202-C, R&D systems Inc., Minneapolis, MN, USA), which was diluted with STD in a 1 : 500 ratio. After blocking each cell at room temperature for 2 hours by adding TBS buffer (10 mM Tris-HCl (Cat. No. T3253, Sigma-Aldrich Co. LLC., Saint Louis, MO, USA) containing 200 µL of blocking buffer (1% BSA (Cat. No. A2153, Sigma-Aldrich Co. LLC., Saint Louis, MO, USA) and 140 mM NaCl (Cat. No. SOD001.1, Bioshop, Canada; pH 7.4) thereto, each well was washed 5 times at room temperature with TBS buffer containing 200 µL of wash buffer (0.05% Tween20; Cat. No. P9416, Sigma-Aldrich Co. LLC., Saint Louis, MO, USA). Continuously diluted WTA, WTA-PGN, and PBS-immunized plasma (50 µL) was added thereto and cultured at room temperature for 2 hours, and mouse reference serum (Cat. No. RS10-101, Bethyl Laboratories, Inc., Montgomery, TX, USA) was used for STD. Each cell was washed 5 times at room temperature with wash buffer (200 µL), treated with 50 µL of goat anti-mouse-IgG (Cat. No. W4021, Promega Corporation, Madison, WI, USA), which was diluted 1 : 2500 and attached to horseradish peroxidase, and incubated at room temperature for 1 hour. The resulting well was washed again 5 times with a wash buffer (200 µL), added with 100 µL of 3,3',5,5'-tetramethylbenzidine (Cat. No. T0440, Sigma-Aldrich Co. LLC., Saint Louis, MO, USA) as a substrate and incubated at room temperature for 10 minutes, treated with 50 µL of 2 N H₂SO₄ (Cat. No. 258105, Sigma-Aldrich Co. LLC., Saint Louis, MO, USA), which is a stop buffer, and the absorbance value measured at 450 nm was amended to the absorbance value measured at 550 nm using the microplate reader (Cat. No. 51119000, Thermo Fisher Scientific Inc., Waltham, MA, USA).

### (iii) Quantitation of bacterial burden in the kidney tissue

The kidney tissue, in which abscess formation was observed, was homogenized with a solution containing 10 mL of 10 mM ethylenediaminetetra acetic acid (EDTA; Cat. No. EDT001.500, Bioshop, Canada) and 0.9% NaCl (Cat. No. 14002, JW Pharmaceutical, Korea). The homogenate was continuously diluted and 50 µL of the diluted homogenate was spread on the sheep blood agar plate (Cat. No. AM601-01, Asan Pharm Co., Ltd., a *rea* gene defect) and cultured in a 37°C incubator (Cat. No. SB-9, EYELA, Japan) for 24 hours and the number of colonies was counted.

### (iv) Pathological observation in the kidney tissue

From the mouse kidney tissue used in the experiment, parts of renal cortex and renal medulla were collected. To confirm the immune cell infiltrates and abscess, which include necrosis of glomerular capsule in the cortex and polymorphonuclear leukocytes, macrophages, and lymphocytes, the tissue slices were subjected to Hematoxylin-eosin (H&E) staining.

That is, the collected tissue was fixed in 10% formalin (Cat. No. HT501128, Sigma-Aldrich Co. LLC., Saint Louis, MO, USA) and then paraffin blocks were prepared. The paraffin blocks were prepared into 3 µm slices and placed on top of slide glass, dried in a 60°C to 70°C oven for one hour, and subjected to H&E staining. The completed slide glass was placed under the optical microscope and the histopathological changes were observed under 200X magnification view.

### Example 6: γδ T cell-mediated in-vivo IL-17A secretion and the protective effect against MRSA infection by purified WTA-PGN derivatives

### <6-1> Induction of cellular immunity by WTA-PGN derivatives

### (6-1-1) When purified WTA-PGN, WTA, and PGN were intraperitoneally injected into a mouse, the mouse secreted IL-17A and IL-1β within 12 hours.

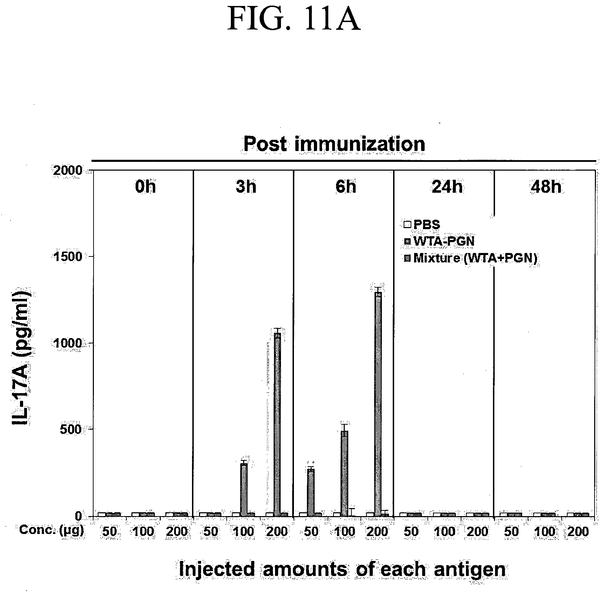

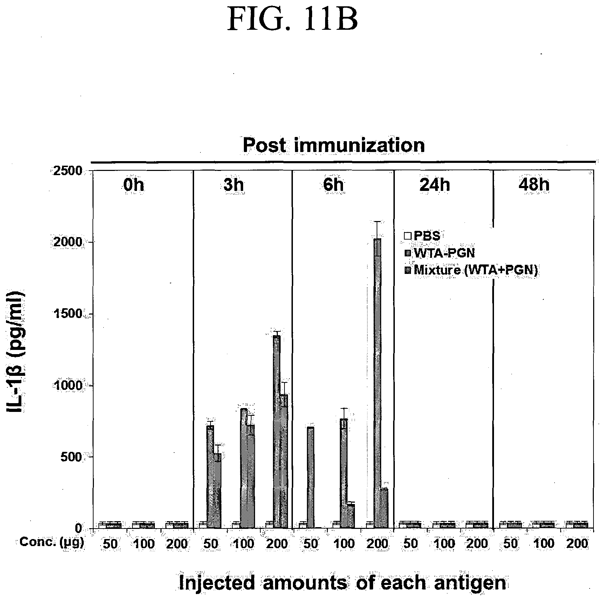

For detailed examination of the physiological activity of the purified WTA-PGN derivatives, the amounts of IL-17A and IL-1β, produced in the peritoneum according to the change in time when the isolated/purified WTA-PGN derivatives at various concentrations (0-, 50-, 100-, and 200 µg/100 µL in PBS) were intraperitoneally injected into a mouse, were observed and the results are shown in FIGS. 11A and 11B, respectively. The temporal epidemiology with respect to the expression of cytokines such as IL-17A and IL-1β were observed by ELISA.

The IL-17A expression started to increase from the time-point of 3 hours after the WTA-PGN injection, was maximally induced in 6 hours, and then rapidly reduced thereafter, and IL-17A was not expressed in 24 hours. When an equal amount of a mixture of WTA and PGN was injected, IL-17A was not produced in all time-points thus confirming that the WTA-PGN structure, in which WTA and PGN are covalently bonded, is necessary for the induction of IL-17A. Additionally, it was confirmed that the IL-17A expression increases in a concentration-dependent manner (FIG. 11A).

The amount of IL-1β expression was also shown to be highest 3 to 6 hours after the injection of WTA-PGN derivatives, and it was reduced to a level of the control in 24 hours. Meanwhile, the mixture of WTA and PGN showed the maximum expression level of IL-1β, 3 hours after the injection, but the IL-1β expression was rapidly reduced, 6 hours after the injection. Like IL-17A, the amount of IL-1β expression was also shown to increase in a concentration-dependent manner (FIG. 11B).

Since it is well known that *Staphylococcus aureus* infection in a particular region of a host can cause to secrete toxins or form biofilms, thereby inhibiting neutrophilmediated phagocytosis, it is suggested that cytokines capable of collecting neutrophils be secreted at the early stage of infection to remove *Staphylococcus aureus* by phagocytosis before the infection is rooted. However, immune modulators which can selectively induce the secretion of IL-17A at the early stage of the infection have not yet been identified.

Due to the basic research results to date, Th17 cells have been known as T cells that can produce IL-17A *in vivo.* However, it has been reported that the continuous secretion of IL-17A by Th17 cells can cause neutrophils to excessively accumulate in a particular region and destroy tissues or organs in a host thereby inducing autoimmune diseases such as lupus, rheumatoid arthritis, etc. Therefore, it is suggested that materials which can induce the IL-17A expression mediated by Th17 cells cannot be used as effective vaccines or immune modulators.

### (6-1-2) The induction of IL-17A and IL-1β at early stage by WTA-PGN derivatives was controlled by IL-10, which is an anti-inflammatory cytokine.

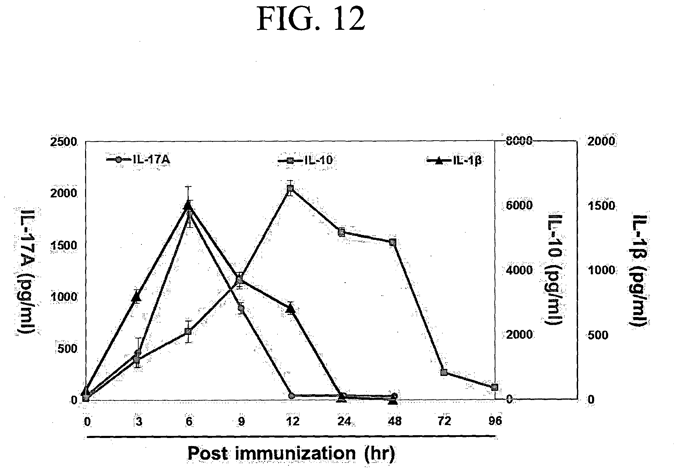

To elucidate why IL-17A and IL-1β are not secreted in a mouse injected with WTA-PGN 12 hours after the injection, the present inventors analyzed the amount of IL-17A, IL-1β, and IL-10 by collecting the peritoneal fluid exudate according to time (0-, 3-, 6-, 9-, 24-, 48-, 72-, and 96 hours) after the injection of the isolated/purified WTA-PGN derivatives (200 µg/100 µL) into a mouse, under the hypothesis that IL-10, which is an anti-inflammatory cytokine, can control these inflammatory cytokines (FIG. 12).

The IL-17A production started to increase 3 hours after the injection of the WTA-PGN derivatives, showed the maximum level in 6 hours, but started to decrease from 9 hours after the injection, and showed no production in 12 hours. IL-1β showed an expression pattern similar to that of IL-17A, but IL-1β expression, after showing the maximum level, 6 hours after the injection, started to decrease and maintained a relatively high level until 12 hours after the injection, but showed no production in 24 hours and thereafter. However, interestingly, after the injection of the WTA-PGN derivatives, IL-10 production gradually increased from 3 hours after the injection, reached the maximum level in 12 hours, started to reduce from 24 hours but maintained at a high level until 48 hours, and started to rapidly reduce thereafter, and almost no IL-10 production was shown in 72 hours and thereafter.

These results led to the expectation that the intraperitoneal injection of WTA-PGN derivatives purified from *Staphylococcus aureus* into a mouse can cause the mouse to secrete inflammatory cytokines such as IL-17A and IL-1β within a short period of time between 3 hours and 6 hours to collect neutrophils thereby capable of removing the *Staphylococcus aureus* around the infection area by phagocytosis. Additionally, the IL-17A secretion by WTA-PGN was shown to be controlled in 12 hours after the injection and thereafter thus confirming that the WTA-PGN derivatives are novel immune modulators which do not induce the loss of tissues or organs in a host due to autoimmune responses by the excess IL-17A secretion.

### (6-1-3) γδT-derived IL-17A was produced by WTA-PGN derivatives

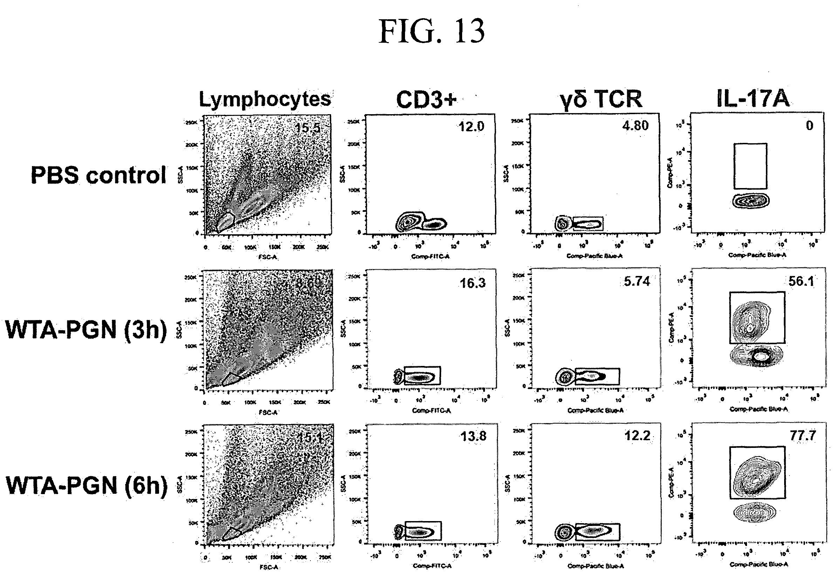

The results of an experiment performed using flow cytometry are shown in FIG. 13, which confirmed that the IL-17A produced when injected with WTA-PGN was the IL-17A derived from γδ T cells. After the WTA-PGN injection, the cells in the peritoneum were collected according to time and CD3+ T cells were collected first. Then, the T cells which had γδT cell receptors (γδ TCR) were collected and the percentage of these T cells over the entire T cells was examined. As a result, 6 hours after the WTA-PGN injection, the percentage increased to 12.2% compared to PBS group. When the IL-17A produced within the γδT cells was quantitated using monoclonal antibody, it was confirmed that, in 3 hours after the injection, 56% of the γδ T cells accumulated in the peritoneum were IL-17A-producing cells, and in 6 hours after the injection, 78% of the γδ T cells accumulated in the peritoneum were IL-17A-producing cells.

These results confirmed that WTA-PGN is a novel immune modulator that can induce the expression of IL-17A selectively mediated by γδT cells.

### (6-1-4) WTA-PGN derivatives failed to induce the IL-17A production derived from CD4⁺ and CD8⁺ T cells.

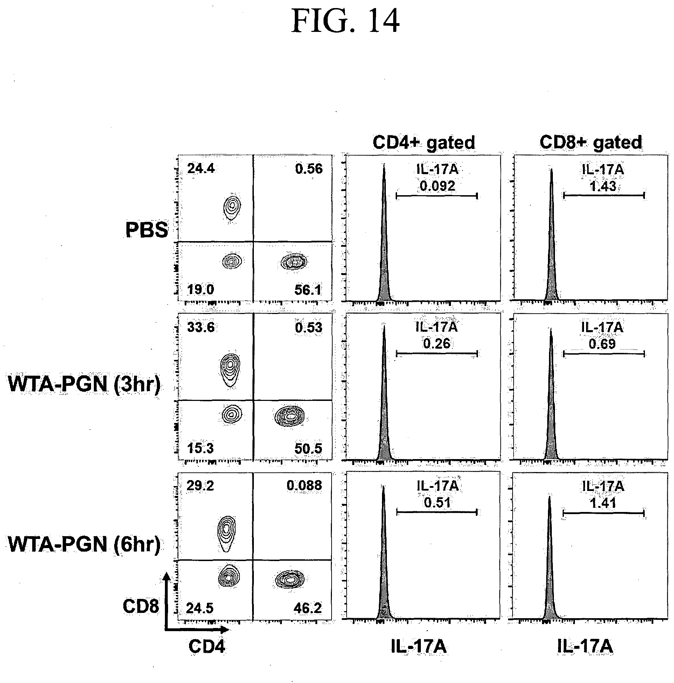

To examine the presence of production of IL-17A derived from CD4⁺- and CD8⁺ T cells by the WTA-PGN derivative injection, the CD4⁺- and CD8⁺ T cells were sorted by flow cytometry and the amount of 1L-17A secreted by these cells was quantitated. The results are shown in FIG. 14.

3 hours and 6 hours after the WTA-PGN injection, the production of IL-17A derived from CD4⁺- and CD8⁺ T cells was not observed. These results confirmed that the WTA-PGN derivatives are novel immune modulators that can control the cellular immune responses by neutrophils by producing only the IL-17A derived from γδT cells, without being involved in the adaptive immunity derived from CD4⁺- and CD8⁺ T cells.

### (6-1-5) WTA-PGN injections failed to induce the IL-17A and IL-1β production in a mouse with the γδ TCR defect.

Since WTA derivatives produced the γδT cell- derived IL-17A, it was examined whether WTA-PGN can induce the expression of IL-17A and IL-1β derived from γδT cells, using a *V*γ2/4^{-/-} mouse, which had a defect in the *Vγ2*/*4* gene (a major subset of γδ TCR), and a wild-type mouse.

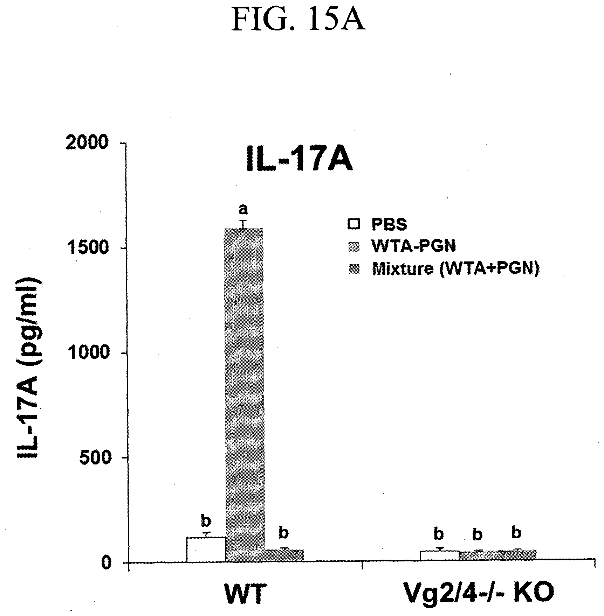

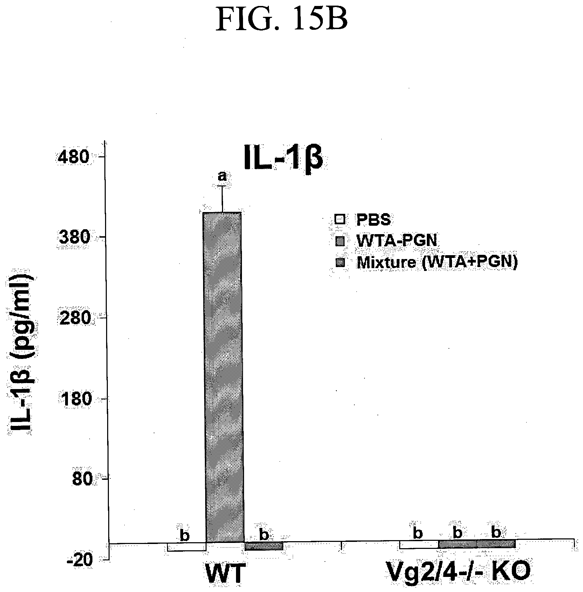

As a result, as shown in FIGS. 15A and 15B, IL-17A and IL-1β were expressed in the group of wild-type mice injected with WTA-PGN as expected, whereas these cytokines were not produced in the group of Vγ2/4^{-/-} mice. When a mixture of WTA and PGN was injected, the expression of IL-17A and IL-1β was observed neither in the wild-type mice group nor in the Vγ2/4^{-/} mice group. From these results, it was confirmed that WTA-PGN can induce the production of IL-17A by activation of Vγ2/4, which is the major subset.

### (6-1-6) Production of memory (memory) γδ T cells by pretreatment of WTA-PGN and the feature of related cytokine expression

### i) Results of examination of cytokine expression pattern after re-infection with MRSA bacteria after 3 pretreatments with WTA-PGN derivatives

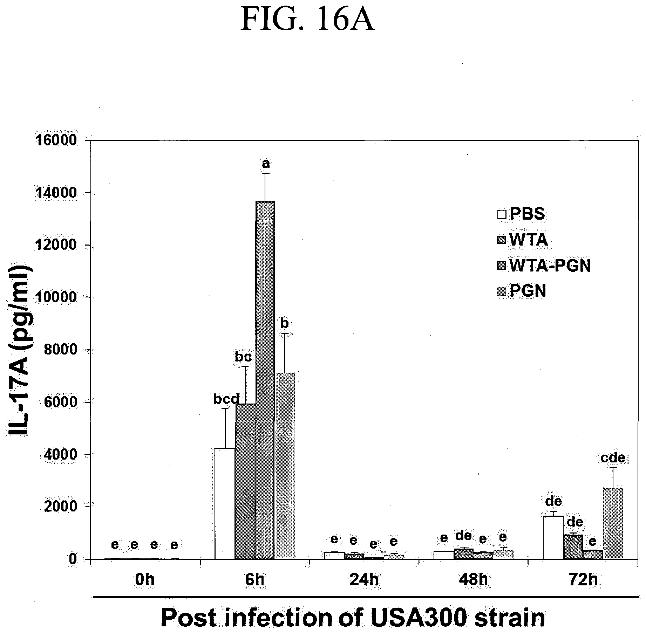

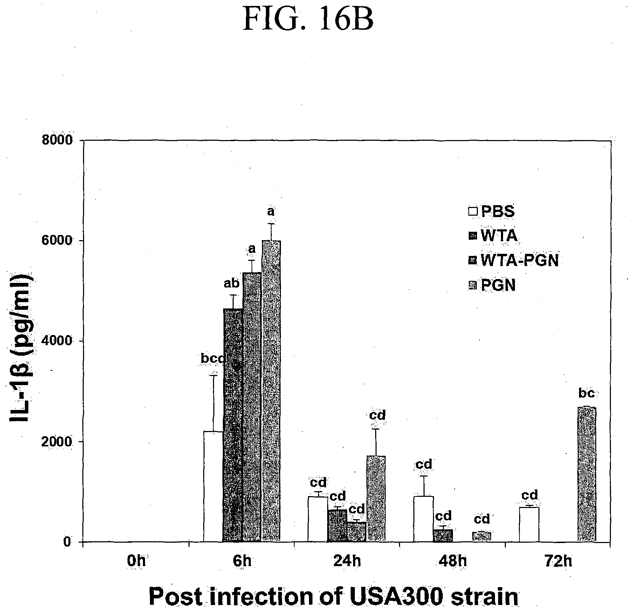

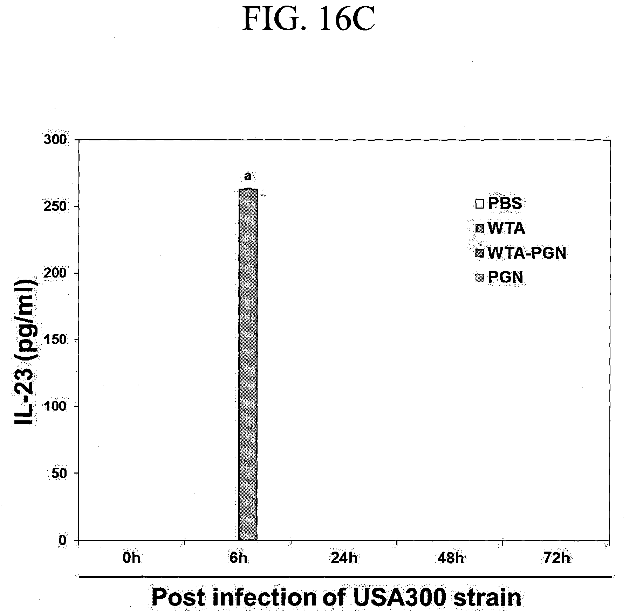

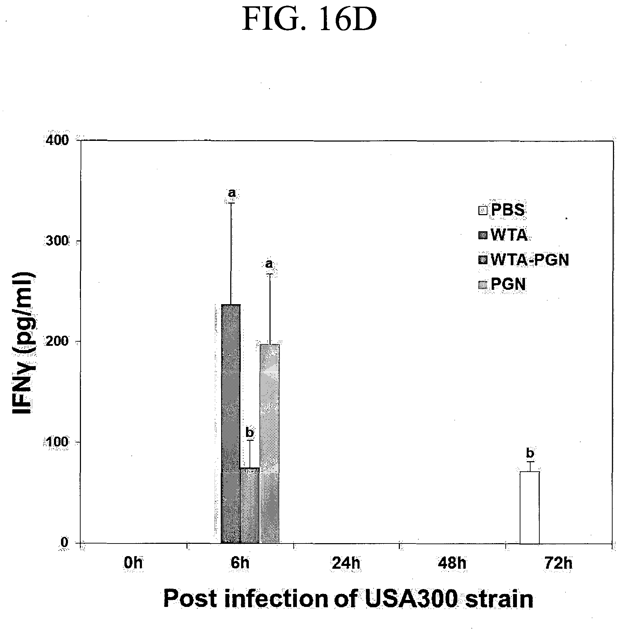

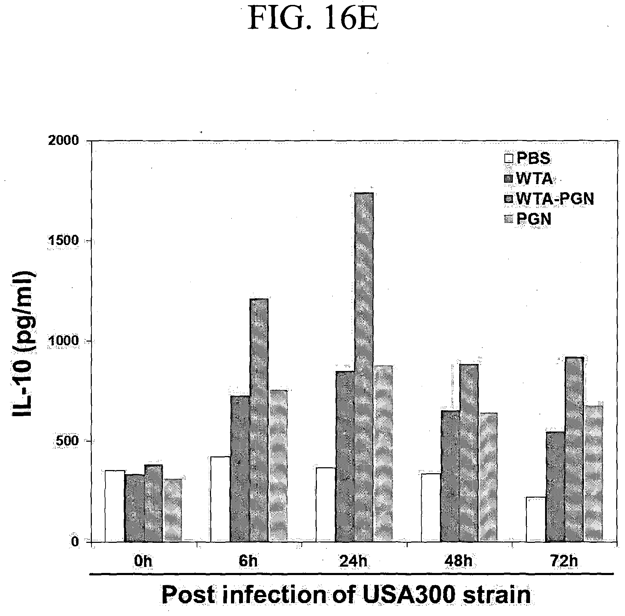

Since the production of memory γδ T cells with respect to the re-infection of *Staphylococcus aureus* is an essential requirement for effective clinical utilization of novel vaccine candidate materials or immune modulators for a new *Staphylococcus aureus* infection, the present inventors examined whether a mouse, which was re-infected in the peritoneum with the *USA300* strain (an MRSA strain) 21 days after three injections of WTA-PGN derivatives at one week intervals, can produce memory γδ T cells (FIG. 16).

First, the changes in inflammatory cytokines and anti-inflamamtory cytokines were examined in a mouse, which was pretreated 3 times with WTA-PGN, WTA, and PGN derivatives. As a result, it was found that the expression level of IL-17A was highest 6 hours after the infection with *USA300* strain, and in 24 hours, the amount of expression was reduced to a level similar to that of the control group (FIG. 16A). IL-17A was expressed in the mouse, which was pretreated 3 times with WTA-PGN, WTA, and a PGN derivative, but the expression level was about a half compared to that of WTA-PGN, and in particular, the 3 times of pretreatment resulted in about a 10-fold increase in the level of IL-17A expression, compared to a single treatment.

The amount of IL-1β production reached the maximum expression level 6 hours after the *USA300* infection, and the expression level was significantly reduced in 24 hours after the infection. The groups pretreated with WTA and PGN, 6 hours after the infection, showed levels of IL-1β expression similar to that of the WTA-PGN group. These results confirmed that the re-infection with *USA300* in the mouse pretreated 3 times with WTA and PGN can exhibit the amount of expression similar to that of WTA-PGN group (FIG. 16B).

Interestingly, the IL-23 production was expressed only in the WTA-PGN group 6 hours after the re-infection with *USA300* strain, and the IL-23 production was not expressed in 24 hours. These results suggest that IL-23 production is closely associated with the IL-17A production mediated by memory γδ T cells (FIG. 16C).

The amount of IFNγ production reached the maximum expression level in a mouse pretreated with WTA and PGN, 6 hours after the infection with *USA300* strain, and the expression level was significantly reduced in 24 hours after the infection. The expression of IFNγ in a mouse pretreated with WTA-PGN was shown to be lowest in the WTA-PGN group, unlike the expression of IL-17A. These results suggest that the IFNγ production is irrelevant to the IL-17A production mediated by memory γδ T cells (FIG. 16D).

Meanwhile, the expression level of IL-10, which is an anti-inflammatory cytokine, significantly increased 3 hours after the infection with *USA300* strain, slightly reduced until 12 hours after the infection, showed the maximum level again in 24 hours, slightly reduced 48 and 72 hours after the infection but was still maintained at high levels. As observed previously, considering that the expression of IL-17A, IL-1β, and IL-23, which are inflammatory cytokines, reached the maximum levels 24 hours after the infection, it was confirmed that IL-10 can effectively inhibit the expression of IL-17A (FIG. 16E).

### ii) The mouse pretreated 3 times with WTA-PGN produced memory γδ T cells which have the CD44^{high}/CD27^{low} marker.

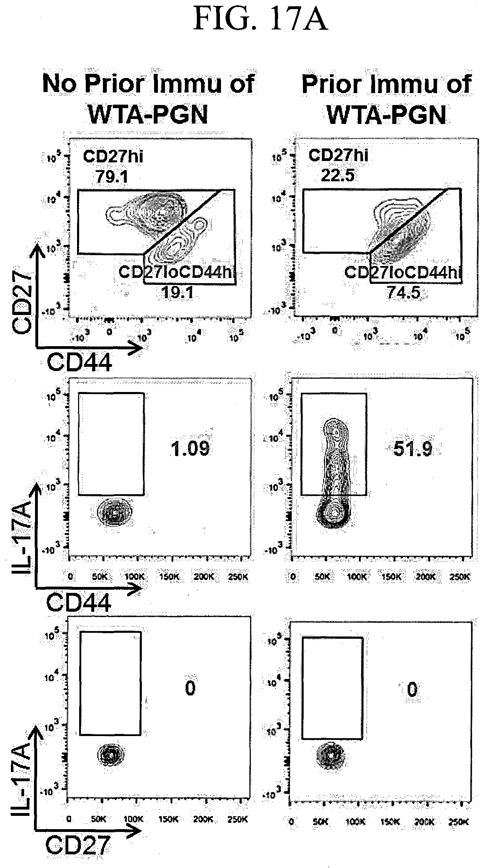

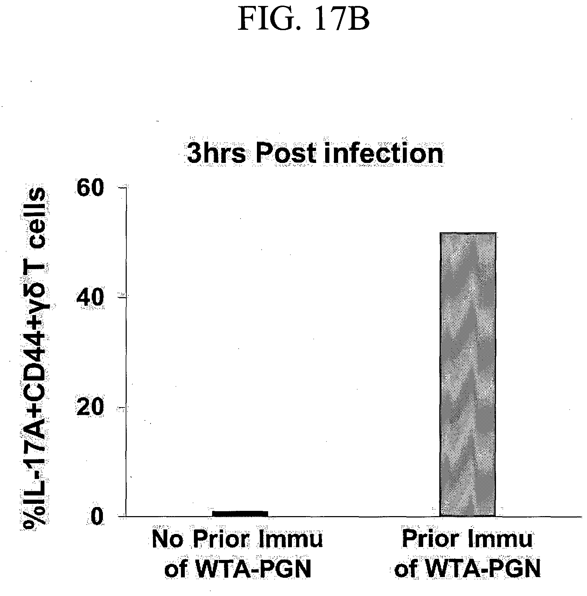

To examine the production of memory γδ T cells in the mouse peritoneum pretreated 3 times with WTA-PGN, the γδ T cells of the mouse pretreated with WTA-PGN were collected 3 hours after the *USA300* infection and the expression of the CD44^{high}/CD27^{low} γδ T cells, which is a marker of memory γδ T cells, was examined. As a result, it was confirmed that the expression level was higher than the group without pretreatment and the memory γδ T cells showed a higher level of IL-17A expression than the control group, whereas the expression of IL-17A was not observed in CD27⁺ γδ T cells (FIGS. 17A and 17B).

### iii) IL-17A production in CD3⁺ γδ T cells

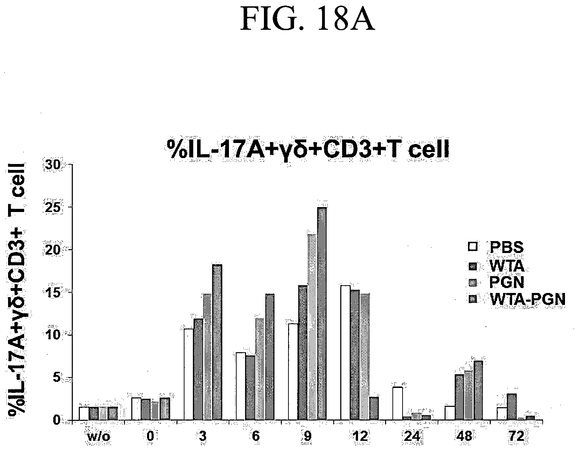

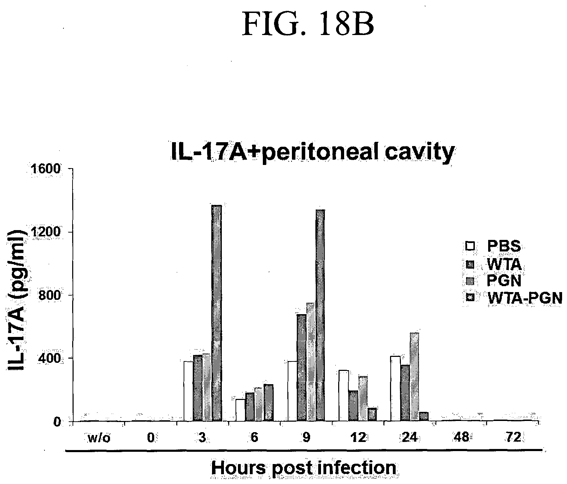

The expression of intracellular and extracellular IL-17A in CD3⁺ γδ T cells was observed by FACS and ELISA, respectively, in the mouse that was pretreated 3 times with WTA-PGN and thereby produced memory γδ T cells, by infecting with *USA300* strain. As a result, the mouse pretreated with WTA-PGN showed a high expression level of IL-17A during the period of 3 hours to 9 hours after the infection (FIGS. 18A and 18B). These results confirm that WTA-PGN can effectively induce memory γδ T cells and the induced memory γδ T cells can act as major cells for producing IL-17A.

### iv) IL-17A production in CD4⁺ and CD8⁺ T cells

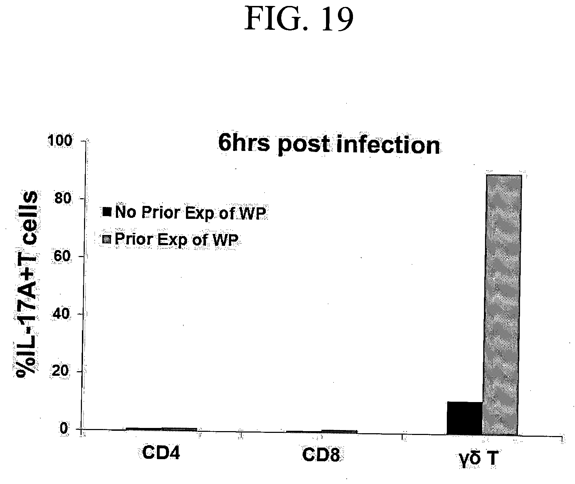

To reconfirm the previous result that the major cells that produce IL-17A after the pretreatment with WTA-PGN are γδ T cells that induce the immune responses, the expression of IL-17A derived from CD4⁺- and CD8⁺ T cells which are involved in adaptive immune responses (FIG. 19). The IL-17A derived from CD3⁺ γδ T cells was produced 90% or higher, but when the expression of IL-17A was examined after isolating the CD4⁺- and CD8⁺ T cells from the mouse pretreated with WTA-PGN, no expression was observed. From these results, it was reconfirmed that when a mouse was infected with MRSA after pretreatment with WTA-PGN 3 times, only the cellular immunity is induced by γδ T cells, instead of adaptive immune response-related cells such as CD4⁺- and CD8⁺ T cells.

### v) Verification of γδ TCR subpopulation differentiated by WTA-PGN pretreatment

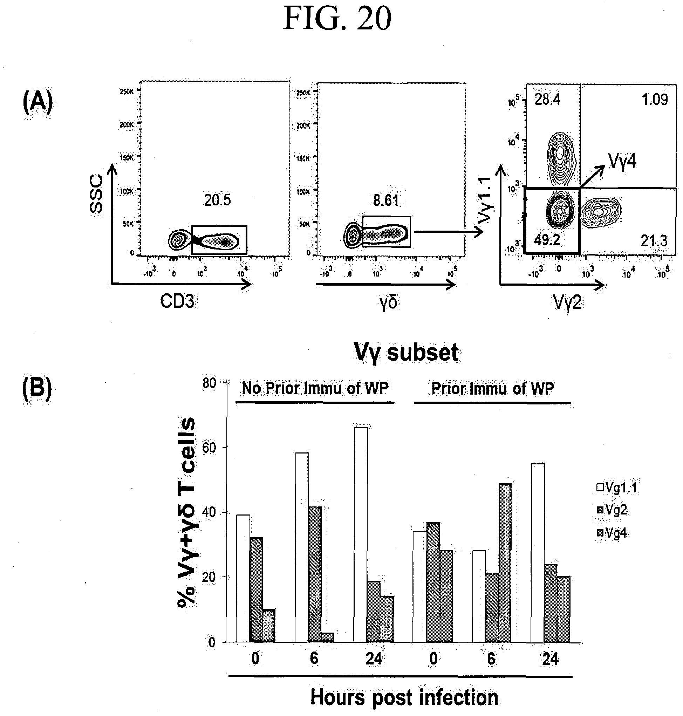

It was examined what subset of the γδ TCR increases when the mouse, pretreated 3 times with WTA-PGN derivatives and thereby having memory γδ T cells produced, was infected with *USA300* strain. As a result, it was confirmed that the Vγ4 subset was expressed highest (FIG. 20). Since anti-Vγ4-Ab is not available in the commercial Vγ4 subset at present, the double negative population region of Vγ1.1 and Vγ2 was confirmed by gating as the Vγ4 population. According to the result of recent studies, at the *USA300* strain infection, the rapid influx of Vγ1.1⁺ and Vγ2⁺ was observed initially, and the shifting of the γδ TCR subset into the Vγ1.1⁺ and Vγ4⁺ γδ T cells was followed. Additionally, with respect to Vγ4⁺ cells, it was reported that when *S. aureus* infection and memory responses were induced continuously, the population was increased even further, and is directly involved in the production of IL-17A (J Immunol 2014; 192: 3697 - 3708).

### vi) IL-23 expression in dendritic cells

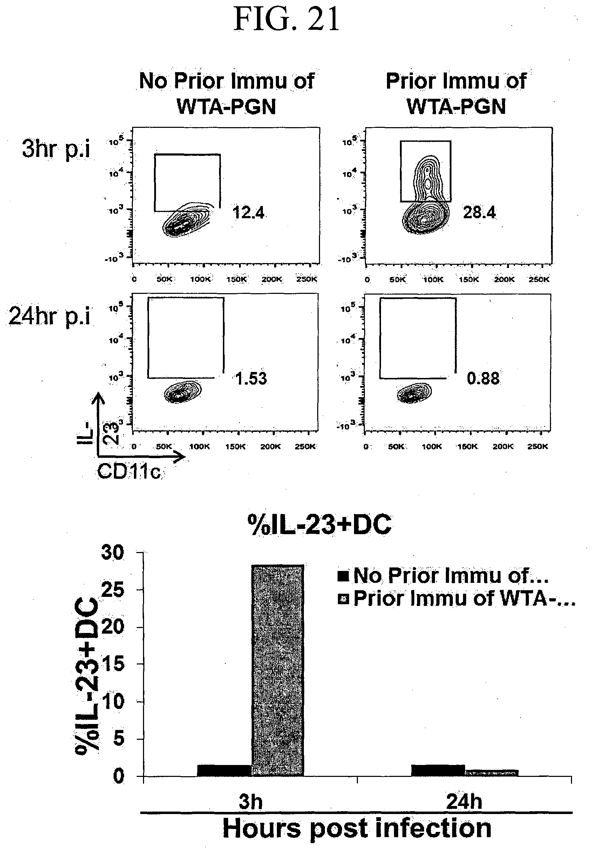

To examine on which cell expresses the IL-23, which was previously shown to be expressed by retreatment with WTA-PGN, the present inventors conducted the studies with a focus on the possibility of dendritic cells (FIG. 21). After pretreating 3 times with WTA-PGN followed by infection with *USA300* strain, the IL-23 expression within the dendritic cells was confirmed. As a result, it was confirmed that the group pretreated with WTA-PGN showed an increase in IL-23 expression compared to the group without pretreatment, however, 24 hours after the infection, no expression was observed. From these results, it was suggested that IL-23 is produced in the dendritic cells at the early stage of the MRSA infection, which triggers γδ T cells to induce IL-17A expression.

### <6-2> Protective effect against MRSA (S. aureus USA300 strain) and MSSA (S. aureus NRS184 strain) infections in vivo by the memory response induced by WTA-PGN immunization

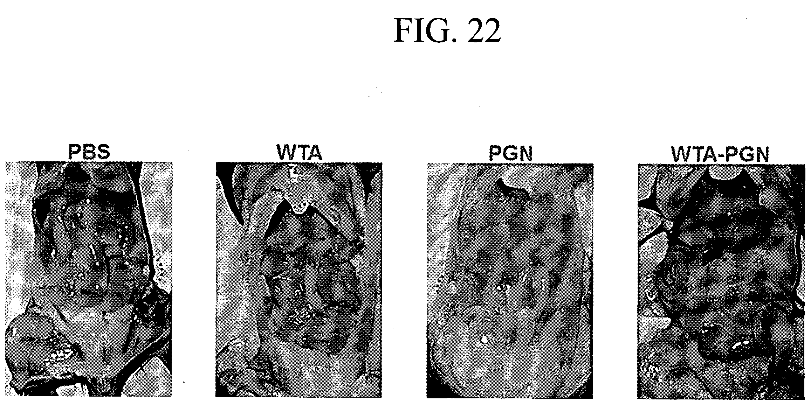

A mouse was immunized 3 times with WTA-PGN intraperitoneally, infected with *USA300* strain (1 x 10⁸ cells) on day 35, and the shapes of organs being produced in the peritoneum and formation of abscess were observed 72 hours after the infection. The results are shown in FIG. 22.

In the control group, abscess was observed in the peritoneum but abscess was not observed at all in the WTA-PGN group, thus suggesting that the IL-17A production mediated by γδ T cells induce the accumulation of neutrophil and increase phagocytosis thereby effectively protecting the host from the *USA300* infection. Meanwhile, although abscess was not observed in the group treated with WTA, there was an abnormal finding in the liver tissue. The group treated with PGN appeared to be in better conditions than the control group but abscess was observed.

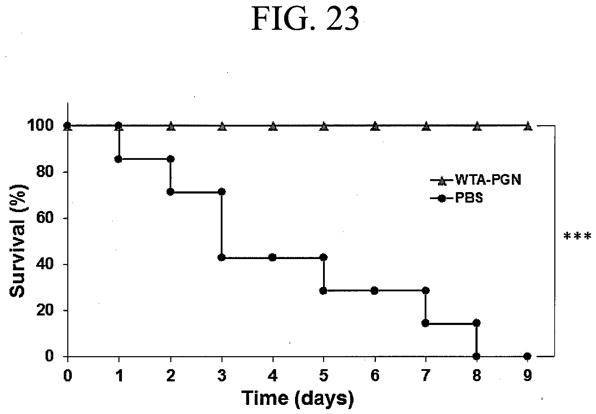

Additionally, to examine the survival rate of the mice, in which a memory response was induced by immunization with WTA-PGN, after infection with *USA300* strain, the mice were immunized 3 times with WTA-PGN via an intraperitoneal injection, infected with *USA300* strain (5 x 10⁸ cells) on day 35, and monitored for 9 days. The results are shown in FIG. 23. All the mice in the group immunized with WTA-PGN survived, whereas all the mice in the PBS group, i.e., the control group, died on day 8. From these results it was suggested that the mice immunized with WTA-PGN can effectively perform an *in-vivo* defense against the MRSA infection at high concentration due to cellular immunity and memory responses at the early stage of the infection.

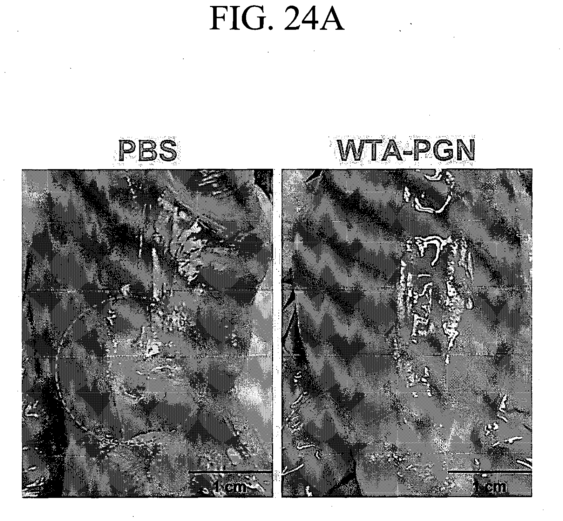

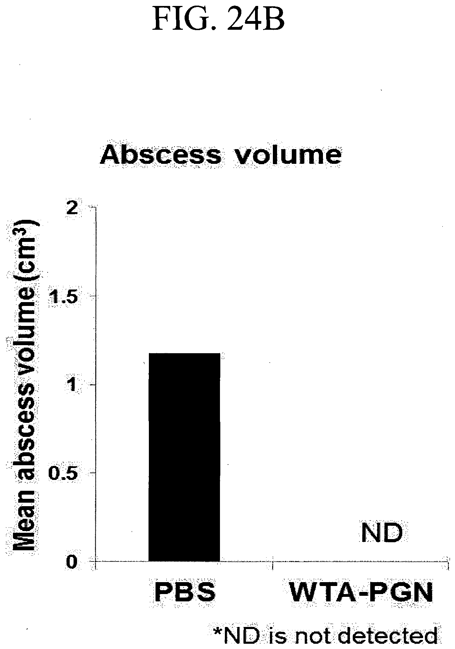

Meanwhile, to examine whether WTA-PGN, which showed a protective effect against the MRSA infection, can also show a protective effect against the MSSA infection, a mouse was immunized 3 times with WTA-PGN via an intraperitoneal injection, infected with *S. aureus* NRS184 strain (5 x 10⁸ cells) on day 35, and the presence of abscess in the peritoneum was observed on day 7 (FIGS. 24A and 24B). As shown in FIG. 24, the presence of abscess was observed in the peritoneum in the PBS group (control) but abscess was not observed at all in the group pretreated with WTA-PGN thus confirming that WTA-PGN can exhibit a strong protective effect against MSSA strain infection as well as against MRSA strain infection.

### <6-3> Protective effect of WTA-PGN immunization in a host against MRSA infection in NZW rabbits and guinea pigs

Since the effect of WTA-PGN immunization on the production of memory γδ T cells and the protective effect against MRSA infection were confirmed in a mouse experimental model, it was examined whether WTA-PGN can exhibit a protective effect against MRSA infection after WTA-PGN immunization in animal models such as NZW rabbits and guinea pigs, instead of a mouse model.

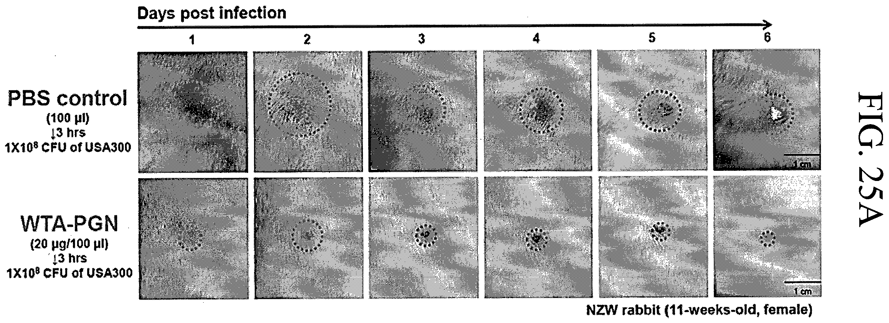

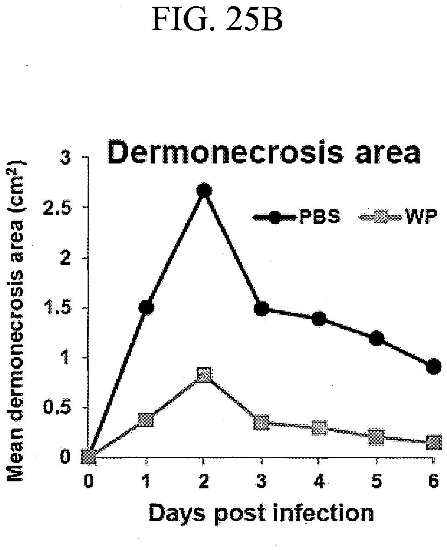

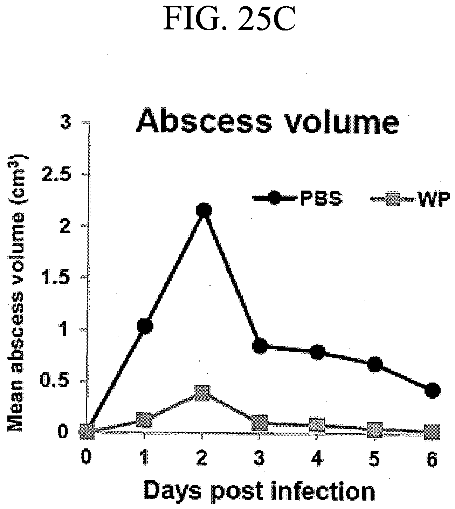

First, the skin of NZW rabbits was immunized with 20 µg of WTA-PGN by subcutaneous injection, and 3 hours after the immunization, infected with MRSA (1 x 10⁸ CFU of *USA300*) by subcutaneous injection, and the protective effect was observed. The results are shown in FIGS. 25A to 25C.

As a result, compared with the control group immunized with PBS (100 µL), the dermonecrosis area and the size of abscess volume of the immunized rabbits were significantly small and rapidly recovered and thus almost no abscess tissue was observed on day 6. From these results, it was confirmed that WTA-PGN induces cellular immunity even in NZW rabbits as well as in a mouse model, and also exhibits a protective effect against MRSA infection.

Additionally, NZW rabbits and guinea pigs models were immunized with WTA-PGN (20 µg WTA-PGN/100 µL PBS) by subcutaneous injection, and 6 hours after the immunization, infected with MRSA (5 x 10⁸ CFU of *USA300*) by subcutaneous injection, and the presence of a protective effect was observed.

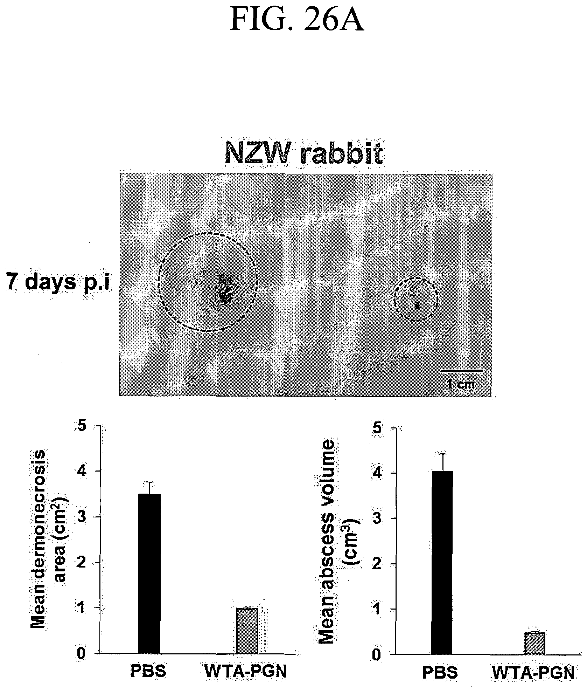

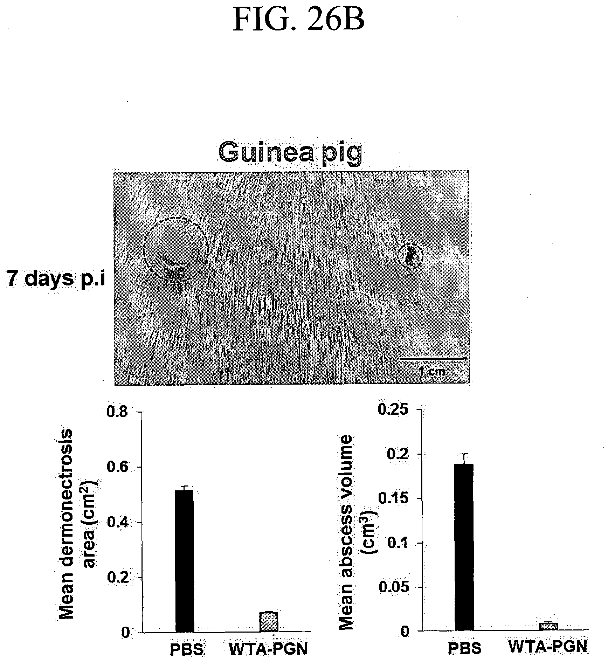

As a result, it was observed that in animals immunized with WTA-PGN (20 µg WTA-PGN/100 µL PBS), both in NZW rabbits and guinea pigs, the size of abscess was shown to be significantly small and also rapidly recovered (FIGS. 26A and 26B). Accordingly, it was confirmed that WTA-PGN has a protective effect against MRSA infection in NZW rabbits and in guinea pigs, which are assumed to have an immune system closest to that of humans, and thus it was confirmed that a host can be protected from MRSA infection by pretreatment with WTA-PGN.

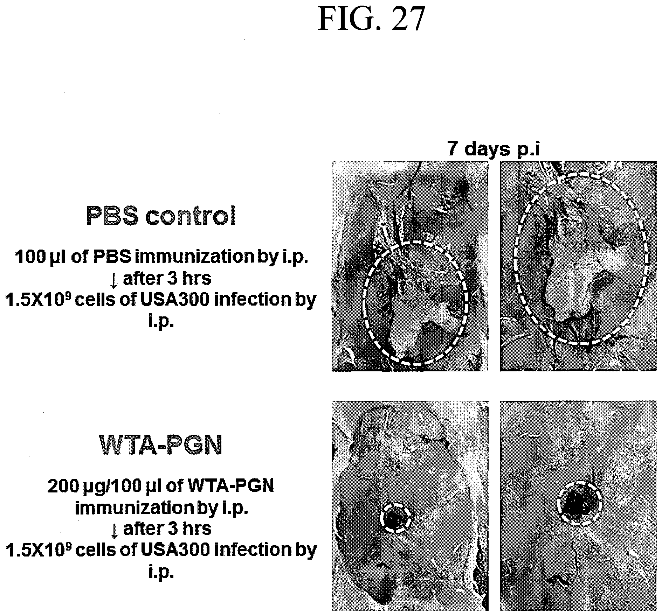

After injecting WTA-PGN into the peritoneum of guinea pigs, for the observation of a protective effect in a host against MRSA infection, the guinea pigs were immunized with PBS and 200 µg of WTA-PGN by intraperitoneal injection, respectively, and 3 hours thereafter, infected with 1.5 x10⁹ CFU of *SA300.* Seven days after the immunization, the animals were dissected and the tissues were observed, and as a result, the size of abscess was significantly reduced in animals immunized with WTA-PGN, compared with the control group immunized with PBS, and there was almost no hemolysis by MRSA (FIG. 27).

From the above results, it was confirmed that WTA-PGN, which showed a protective effect against MRSA infection by pretreatment of the peritoneum of a mouse with WTA-PGN, also has a protective effect in a model system using the peritoneum of guinea pigs against MRSA infection.

### <6-4> IL-17A and IL-1β production by WTA-PGN immunization in a wild-type mouse, a mouse with TLR-9 gene defect, and a mouse with Caspase-1 gene defect

For the study of signal transduction mechanism as to how WTA-PGN produces IL-17A derived from γδ T cells, studies were performed on toll-like receptors (TLR) pathway and inflammasome pathway, which are known to be involved in the induction of IL-23 and IL-1β expression in dendritic cells, respectively.

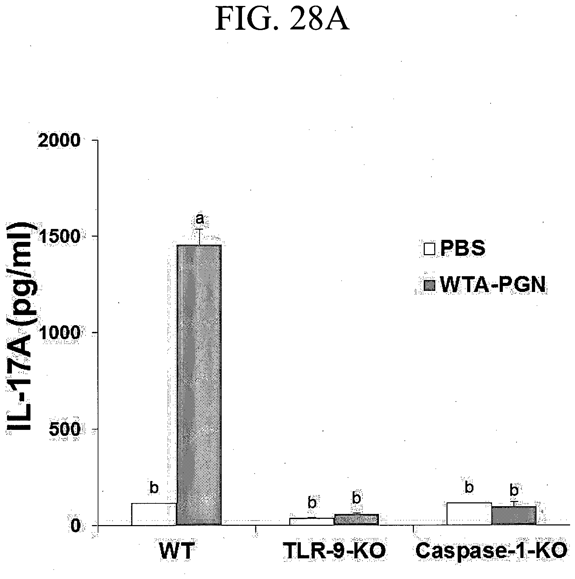

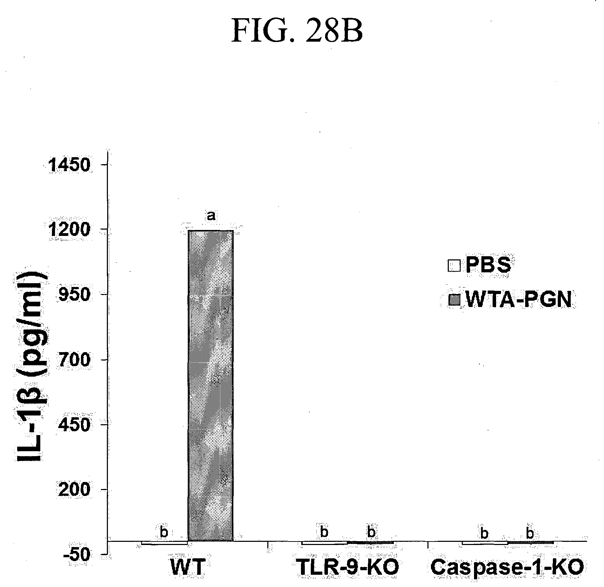

The WTA- PGN isolated from *ΔoatAΔlgt* and the WTA-PGN isolated from a wild-type bacteria were injected into a wild-type mouse, a mouse with a defect in the *TLR-9* gene, and a mouse with a defect in the *Caspase-1* gene, and 6 hours after the immunization, the IL-17A production was measured in a peritoneal fluid exudate. As a result, it was confirmed that IL-17A and IL-1β were expressed only in the group of mice injected with the WTA-PGN isolated from *ΔoatAΔlgt* and these cytokines were not produced in the mouse with a defect in the *TLR-9* gene, and the mouse with a defect in the *Caspase-1* gene (FIGS. 28A and 28B).

These results suggested that TLR-9 pathway and inflammasome pathway will be involved when the WTA-PGN isolated from *ΔoatAΔlgt* produces IL-17A derived from γδ T cells.

### <6-5> Comparison of expression features of cytokines and chemokines induced after WTA-PGN pretreatment in macrophages, dendritic cells, and γδ T cells

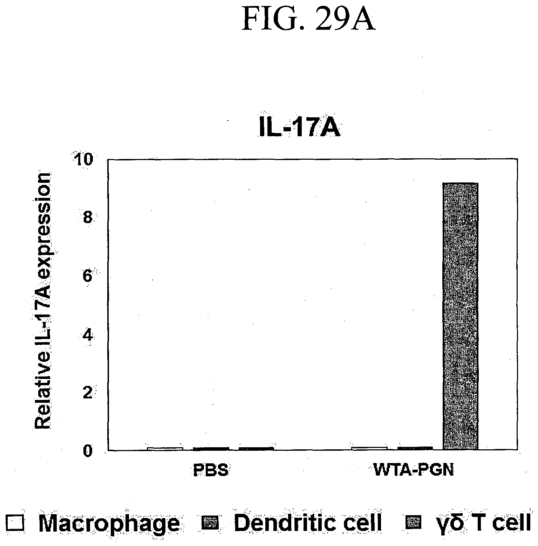

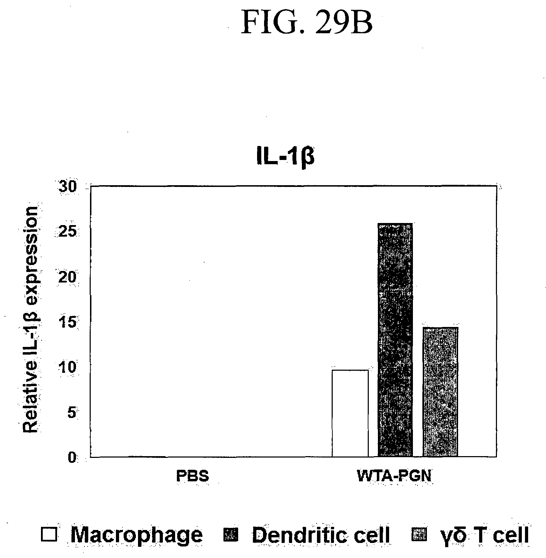

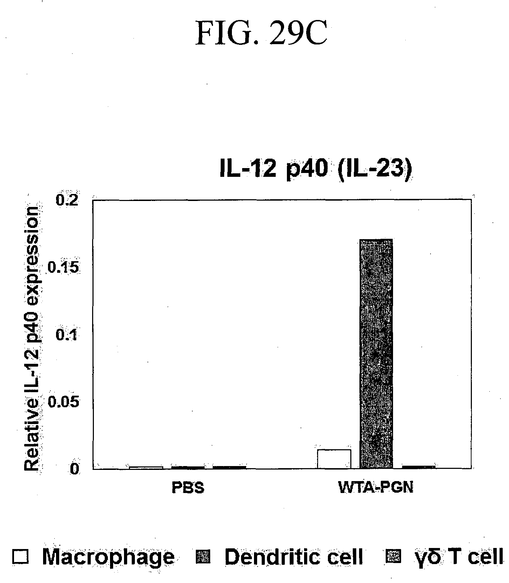

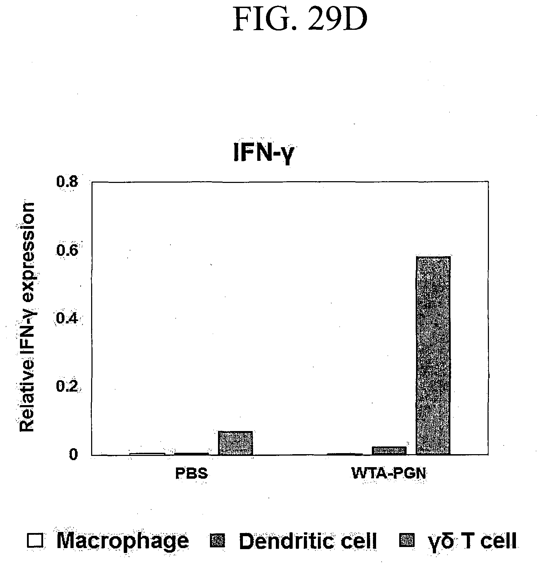

To understand the high level signal transduction system necessary for the γδ T cell-dependent IL-17A secretion and the biological function and mechanism of WTA-PGN, macrophages, dendritic cells, and γδ T cells were separated from the peritonial exudate cells (PEC) and the cytokines induced from these cells were examined (FIG. 29).

As a result, it was confirmed that, with WTA-PGN immunization, IL-17A is expressed in γδ T cells, IL-1β is expressed in dendritic cells and γδ T cells, IL-12 p40 (IL-23) is expressed in dendritic cells, and IFNγ is expressed in γδ T cells, respectively.

Recently, there are growing number of reports confirming that IL-23 and IL-1β expression in dendritic cells have an important role in the production of IL-17A in non-classical T cells, i.e., γδ T cells. Therefore, it was determined that there is a need for a more specific study as to how the cytokines recognize ligands and interact with each other, with the WTA-PGN immunization.

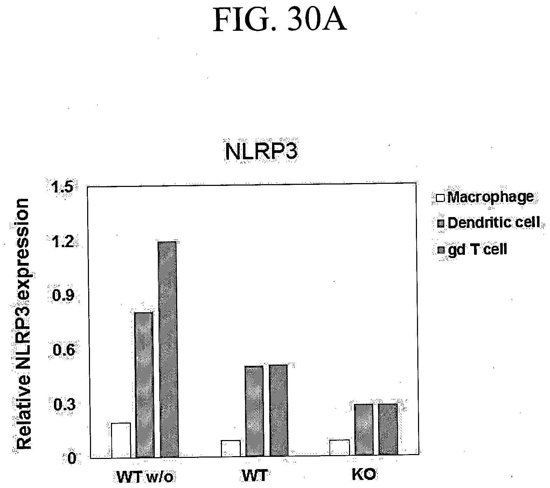

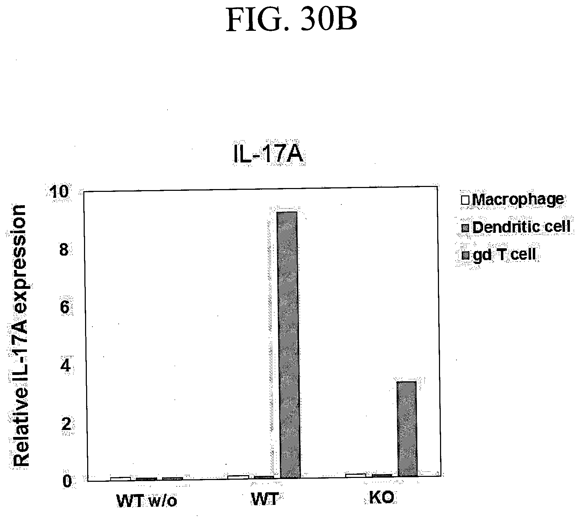

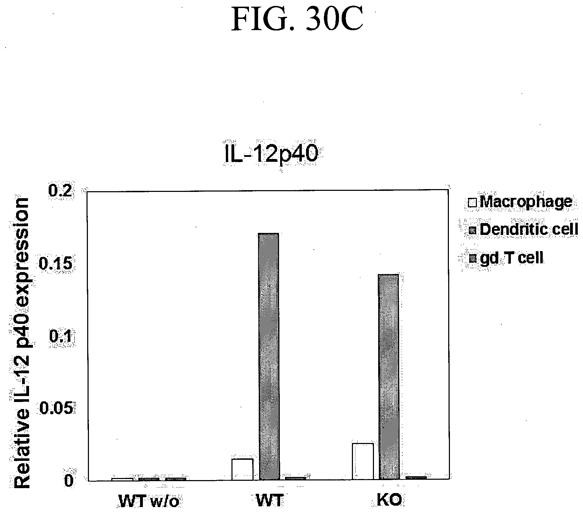

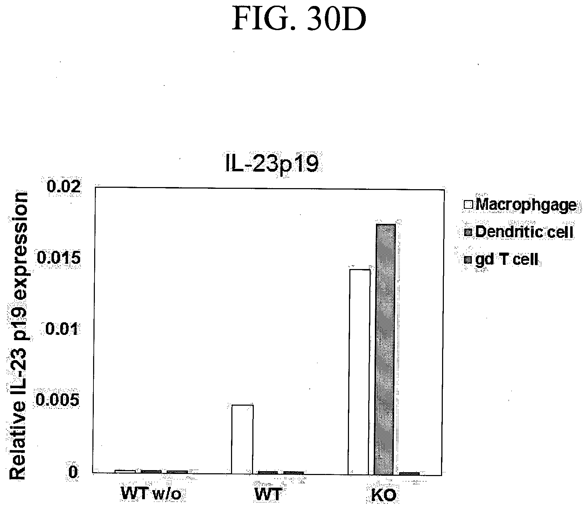

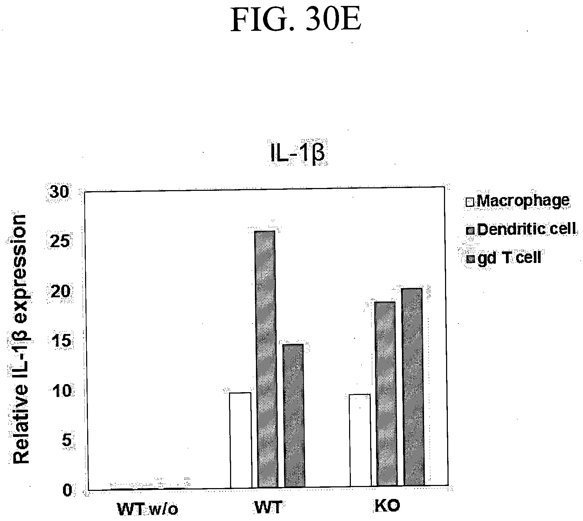

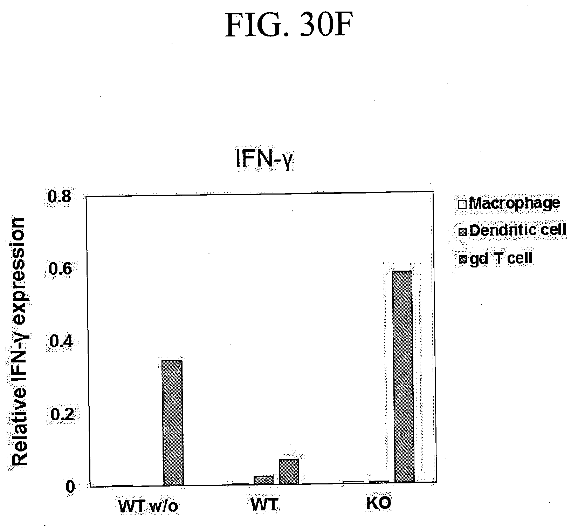

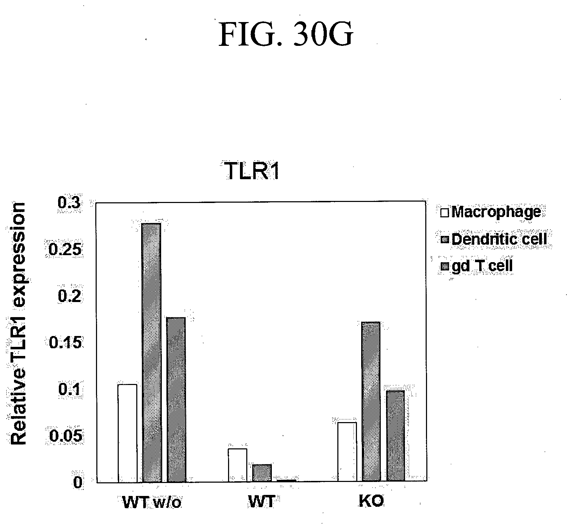

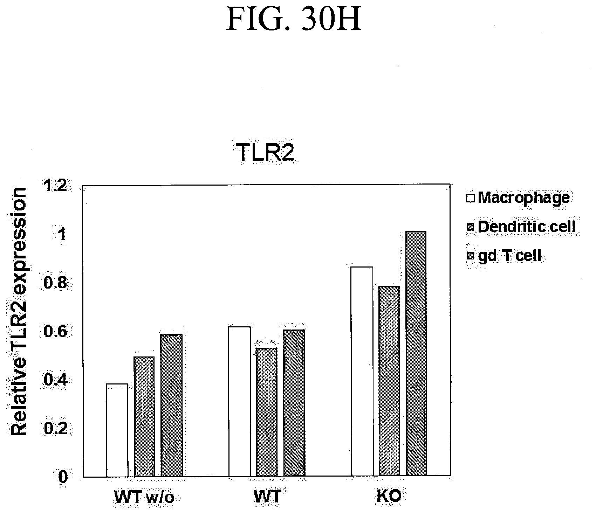

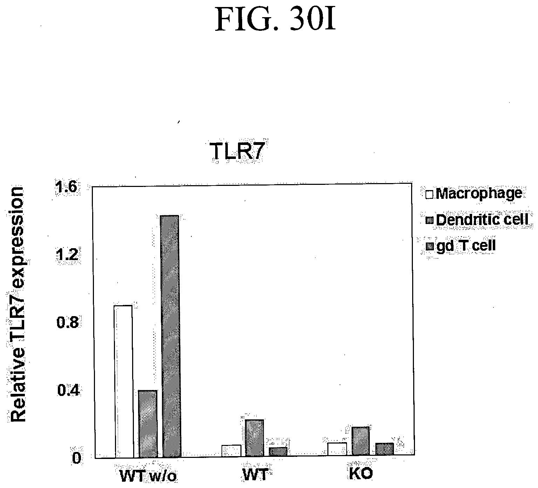

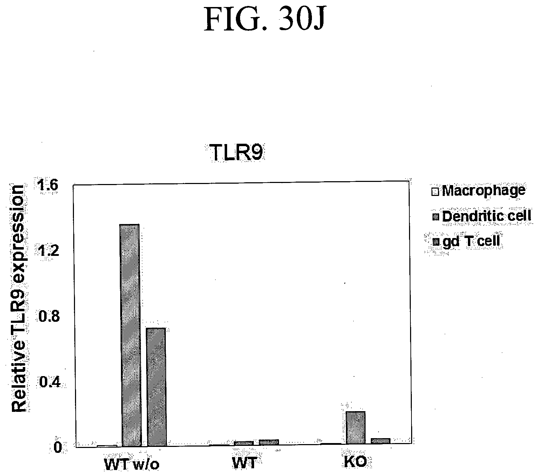

Additionally, WTA-PGN immunization was performed using a wild-type mouse and a mouse with a defect in *NLRP3* gene, and macrophages, dendritic cells, and γδ T cells were separated, and the expression state of *NLRP3,* cytokine, and *TLR1* to *TLR9* genes were examined by qRT-PCR. As a result, it was confirmed that *NLRP3* was mainly produced in dendritic cells and γδ T cells, highly expressed in the control group with no treatment, and slightly reduced in the wild-type mouse and the mouse with a defect in *NLRP3* gene, however, the expression level was higher in the wild-type mouse compared to that of the mouse with a defect in *NLRP3* gene (FIG. 30).

Generally, *NLRP3* is known to be expressed in inflammasome within dendritic cells thereby influencing on the production of IL-1β. Considering the result that the IL-1β expression was shown high in both dendritic cells and γδ T cells in the previous experiment and that the level of *NLRP3* gene expression was almost the same in dendritic cells and γδ T cells, it is suggested that the inflammasome, in which *NLRP3* is involved, may be also present in γδ T cells, and these are assumed to be new findings which have never been reported previously (FIG. 30A).

While IL-17A was not expressed at all in the control group, the level of IL-17A expression upon WTA-PGN immunization increased only in the γδ T cells, which were obtained from a wild-type mouse and a mouse with a defect in *NLRP3* gene, and the level of IL-17A expression was higher in the wild-type mouse compared to the mice group with a defect in *NLRP3* gene. These results suggest that, in the case of the mouse with a defect in *NLRP3* gene, IL-1β production is inhibited within inflammasome of dendritic cells and thus cannot stimulate γδ T cells thereby reducing the level of IL-17A expression (FIG. 30B).

IL-23 is in the form of a heterodimer consisting of *IL-12p40* and *IL-23p19.* Upon examination of the expression of these genes, *IL-12p40* was highly expressed in a wild-type mouse with WTA-PGN immunization and *IL-23p19* was highly expressed in the macrophages and dendritic cells of the mouse with a defect in *NLRP3* gene, however, the level of *IL-23p19* expression was negligible compared to that of *IL-12p40* expression (FIGS. 30C and 30D).

Although IL-1β was not expressed at all in the control group, with WTA-PGN immunization, the level of IL-1β expression was shown high in the group of wild-type mice in the order of dendritic cells> γδ T cells > macrophages, mice, and shown high in the mouse with a defect in *NLRP3* gene in the order of γδ T cells > dendritic cells> macrophages (FIG. 30E). IFNγ expression was significantly inhibited in the group of wild-type mice immunized with WTA-PGN, but highly expressed in the γδ T cells of the control group and the mouse with a defect in *NLRP3* gene (FIG. 30F).

From the above results, it was suggested that there will be a close relation between the IL-17A expression in the γδ T cells and the IL-23 expression in the dendritic cells, whereas the IFNγ expression and the IL-17A expression in the γδ T cells are in the contradictory relationship with each other.

Additionally, to understand the high class signal transduction systeme necessary for the γδ T cell-dependent IL-17A secretion, the expression of *TLR1* to *TLR9* genes were confirmed. *TLR3, -4, -5, and* -*6* genes were not confirmed, and even in the cases of *TLR1, -2, -7, and -9* genes, no significant difference was observed between the control group, the group of wild-type mice with WTA-PGN immunization, and the group of mice having a defect in *NLRP3* gene (FIGS. 30G to 30J). In particular, in the case of WTA-PGN, which was isolated from a *ΔoatAΔlgt* mutant, it was expected to be recognized by *TLR9* present in endosome and thereby secret IL-23. However, as shown in FIGS. above, when a wild-type was immunized with WTA-PGN, *TLR9* gene was almost not shown, and thus another higher class signal transduction system was thought to be present.

### Example 7: Humoral immunity by WTA-PGN derivatives

### <7-1> Subcutaneous immunization by WTA and WTA-PGN produced anti-WTA-IgG in a mouse model.

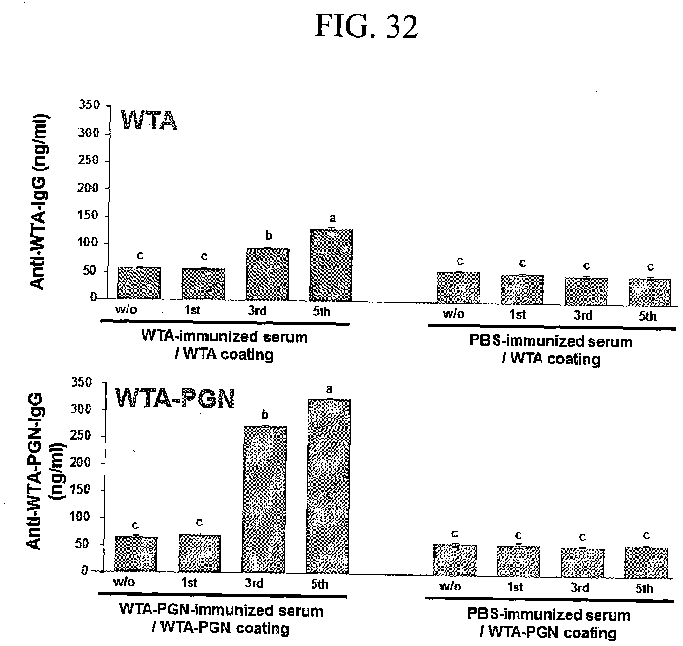

After the immunization with WTA and WTA-PGN, to confirm the production of each antigen-specific antibody, a 96-well plate was coated with WTA and WTA-PGN, and the amount of anti-WTA-IgG and anti-WTA-PGN-IgG production was titrated using an immunized mouse serum (FIG. 32).

As a result, in the case of WTA, when it was immunized 5 times, the amount of anti-WTA-IgG in the blood was increased by about a 3-fold. In contrast, in the case of WTA-PGN, when it was immunized 3 times, the amount of anti-WTA-PGN-IgG was significantly increased by about 5-fold (FIG. 32).

These results enabled an expectation that the immunization with WTA-PGN derivatives will produce antigen-specific antibodies and activate the classical complement pathway and induce opsonophagocytosis thereby providing a protective effect. Therefore, the mouse immunized 5 times with WTA and WTA-PGN was infected with MRSA strain through the caudal vein, and the protective effect in a host was examined.

### <7-2> Change in body weight by MRSA infection after immunization by subcutaneous injection of WTA-PGN derivatives

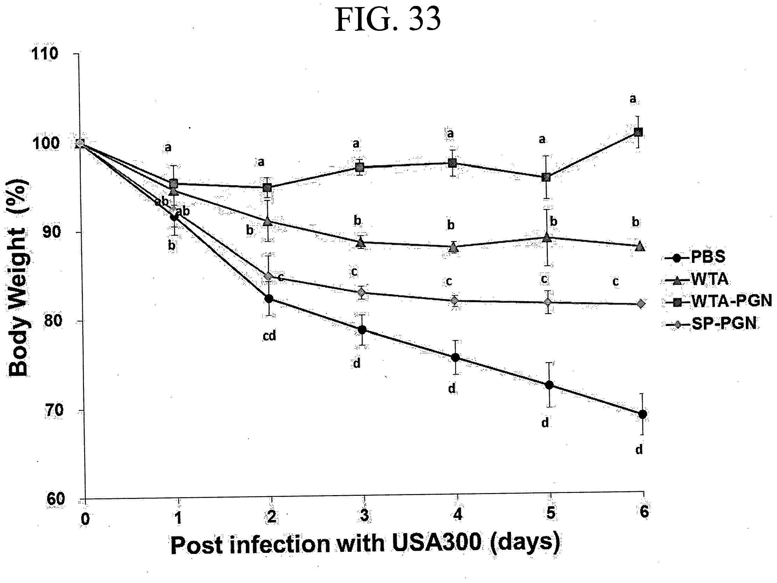

After the immunization with WTA-PGN derivatives 5 times, the animals were infected with *USA300* strain (1 x 10⁷ CFU) on day 70, and the results of monitoring the body weight for a week are shown in FIG. 33.

The group injected with PBS, after the infection, showed a decrease of body weight of more than 15% on day 2 and continuously maintained low body weight. In contrast, the WTA- and PGN groups showed a lower reduction rate in body weight compared to the PBS group, and the WTA-PGN group initially showed a 5% of body weight decrease but recovered its original body weight on day 6.

The above results suggest that, with MRSA infection, the opsonophagocytosis is induced by the production of antigen-specific antibodies, and the mouse protective effect was exhibited by removing bacteria during the early stage of the infection.

### <7-3> MRSA protective effect in a host by WTA-PGN immunization

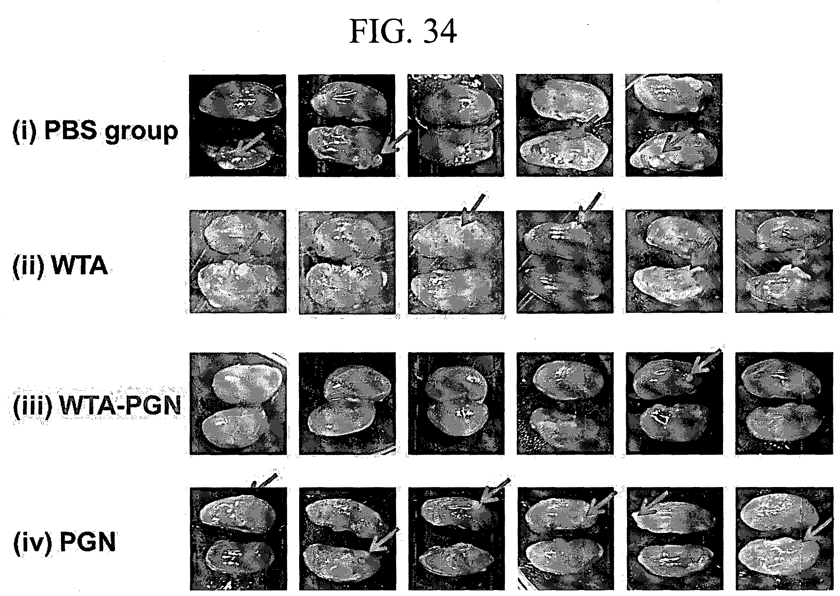

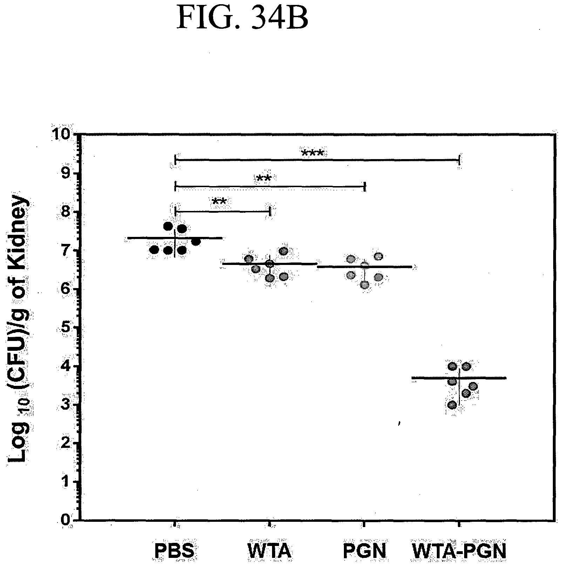

After immunizing mice with_WTA, PGN, and WTA-PGN derivatives isolated from the cell wall of *Staphylococcus aureus,* the mice were infected by intravenous injection of *USA300* strain on day 70. On day 77, the kidney was isolated and the CFU of *USA300* present in the kidneys of the mice and the presence of abscess formation were examined. The results are shown in FIGS. 34A and 34B.

As a result of the observation of the presence of abscess formation in the kidneys of the mice, it was confirmed that the WTA-PGN group showed a significant inhibition on abscess formation compared to the PBS control group as seen in FIG. 34A. When the kidneys were ground and the CUF of MRSA strain present in the kidneys were calculated (FIG. 34B), as expected, the WTA-PGN group was assumed to be a material that acts as an antigen having a protective effect in a mouse against MRSA infection.

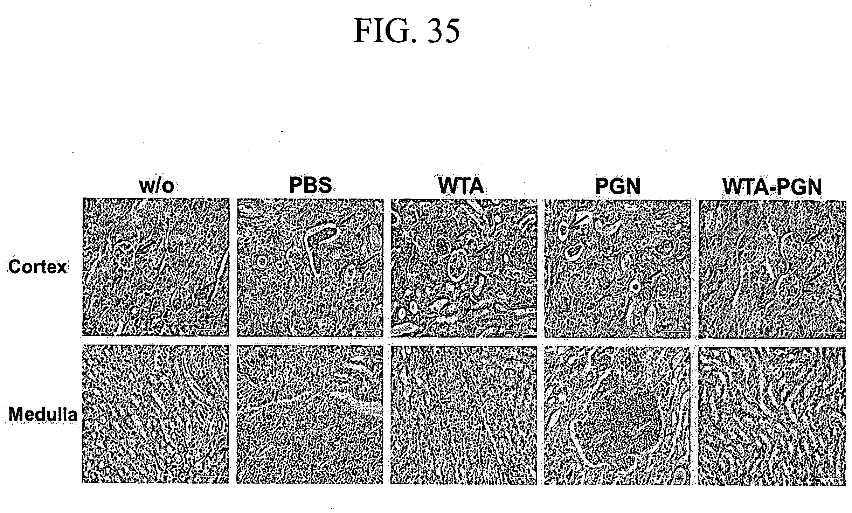

Meanwhile, the results of observation on the histopathological phenomena of a mouse kidney are shown in FIG. 35. The necrosis of glomerular capsule was confirmed in the renal cortex, whereas abscess and the immune cell exudate containing polymorphonuclear leukocytes, macrophages, and lymphocytes were confirmed in the renal medulla. In the case of the cortex, the glomerular capsule in the group of mice immunized with WTA-PGN, was maintained in a shape similar to that of the mice of the control group, whereas necrosis of glomerular capsule was observed in the group of mice immunized with PBS and PGN. In the case of the medulla, a small amount of immune cell exudate was observed in the group of mice immunized with WTA-PGN and a feature almost similar to that of the mice of the control group was shown but no abscess was not found at all which showes a pattern close to that of the normal tissue. Meanwhile, large abscess was observed in the PBS group and the PGN group, and the degree of renal damage was reduced in the order of groups of WTA-PGN> WTA> PGN> PBS, both in the cortex and medulla.

## Claims

1. A composition for preventing or treating *Staphylococcus aureus* infectious diseases comprising wall teichoic acid-attached peptidoglycan (WTA-PGN) as an active ingredient.

2. The composition of claim 1, wherein the WTA-PGN is represented by General Formula 1 below: 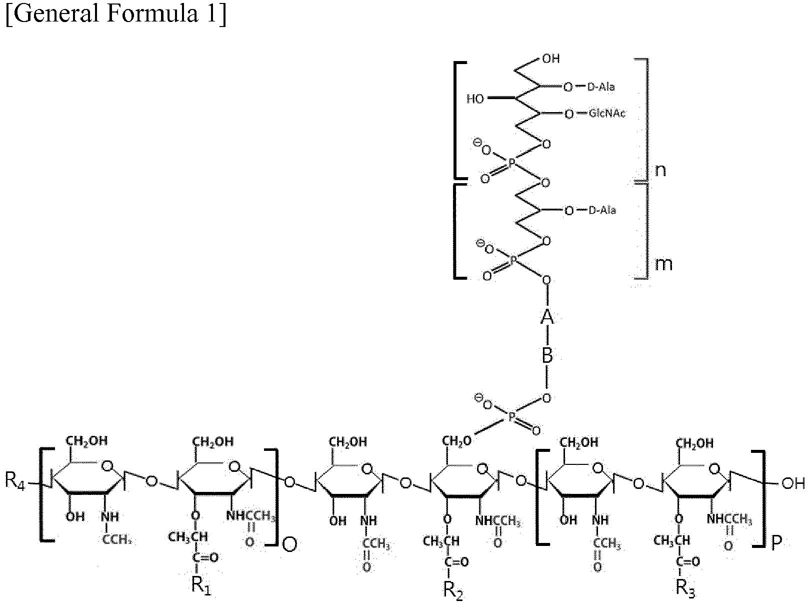 wherein n is an integer of 10 to 50; m is an integer of 1 to 3; A is *N-*acetylmannosamine (ManNAc); B is *N*-acetylglucosamine (GlcNAc); O and P are each independently an integer of 0 to 5; R₁ to R₃ are each independently hydroxy, tetrapeptide or pentapeptide; and R₄ is hydroxy or *N*-acetylmuramic acid (MurNAc).

3. The composition of claim 2, wherein A and B are connected by a β-position with each other.

4. The composition of claim 2, wherein n is an integer of 35 to 45; m is 3; A is *N-*acetylmannosamine (ManNAc); B is N-acetylglucosamine (GlcNAc); O and P are each independently an integer of 0 to 5; R₁ to R₃ are each independently hydroxy, tetrapeptide or pentapeptide; and R₄ is hydroxy or *N*-acetylmuramic acid (MurNAc).

5. The composition of claim 4, wherein n is 40; m is 3; A is *N*-acetylmannosamine (ManNAc); B is *N*-acetylglucosamine (GlcNAc); O and P are each independently an integer of 0 to 5; R₁ and R₂ are each independently tetrapeptide; R₃ is hydroxy, tetrapeptide or pentapeptide; and R₄ is hydroxy or N-acetylmuramic acid (MurNAc).

6. The composition of claim 5, wherein the tetrapeptide is -A₁-A₂-A₃-A₄, wherein A₁ is Ala or Gly, A₂ is Glu or Asp, A₃ is Lys, Arg or His, and A₄ is Ala or Gly.

7. The composition of claim 5, wherein the tetrapeptide is -(L-Ala)-(D-Glu)-(L-Lys)-(D-Ala).

8. The composition of claim 1, wherein the *Staphylococcus aureus* is methicillin-resistant *Staphylococcus aureus* (MRSA), methicillin-sensitive *Staphylococcus aureus* (MSSA), or pathogenic *Staphylococcus aureus.*

9. The composition of claim 1, wherein the *Staphylococcus aureus* infectious disease is selected from the group consisting of soft tissue infection, pyogenic arthritis, pyogenic osteomyelitis, otitis media, pneumonia, sepsis, acute respiratory tract infection, catheter-related infection, postoperative infection, bacteremia, endocarditis, and food poisoning.

10. A method for preventing or treating *Staphylococcus aureus* infectious diseases in a subject comprising administering the composition of any of claims 1 to 9 to a subject in need thereof.

11. The method of claim 10, wherein the method simultaneously induces opsonophagocytosis and phagocytosis.

12. The method of claim 10, wherein the method increases the number of γδ-T cells, the amount of IL-17A production and the amount of IL-1β production in the subject, within 24 hours after the composition is administered to the subject.

13. The method of claim 10, wherein the method increases the amount of IL-10 production in the subject, 12 hours after the composition is administered.

14. A method for preparing a soluble wall teichoic acid-attached peptidoglycan (WTA-PGN), comprising the steps of:
(1) obtaining a double mutant strain in which lipoprotein diacylglycerol transferase (*lgt*) and *O*-acetyl transferase (*oatA*) genes are deleted from a wild-type *Staphylococcus aureus*;
(2) disrupting the strain with a double mutation and obtaining an insoluble WTA-PGN from the disrupted strain;
(3) treating the insoluble WTA-PGN with a β-lytic enzyme;
(4) obtaining a fraction comprising a soluble WTA-PGN from the enzyme-treated product in Step (3);
(5) treating the fraction comprising a soluble WTA-PGN with a lysozyme or a mutanolysin; and
(6) obtaining a soluble WTA-PGN from the enzyme-treated product in Step (5).

15. The method of claim 14, further comprising the step of purifying the soluble WTA-PGN after Step (6).


<110>	Green Cross Corporation
	Pusan National University Industry-University Cooperation Foundation

<120>	Composition for preventing or treating staphylococcus aureus
	infection

<130>	PCB405132MOG

<150>	US 62/004,564
<151>	2014-05-29

<150>	US 62/040,452
<151>	2014-08-22

<150>	US 62/044,196
<151>	2014-08-30

<160>	34

<170>	KoPatentIn 3.0

<210>	1
<211>	21
<212>	DNA
<213>	Artificial Sequence

<220>
<223>	Forward primer for HPRT

<400>	1
ttatggacag gactgaaaga c	21

<210>	2
<211>	22
<212>	DNA
<213>	Artificial Sequence

<220>
<223>	Reverse primer for HPRT

<400>	2
gctttaatgt aatccagcag gt	22

<210>	3
<211>	23
<212>	DNA
<213>	Artificial Sequence

<220>
<223>	Forward primer for IFN-gamma

<400>	3
gagccagatt atctctttct acc	23

<210>	4
<211>	20
<212>	DNA
<213>	Artificial Sequence

<220>
<223>	Reverse primer for IFN-gamma

<400>    4
gttgttgacc tcaaacttgg                                                    20

<210>    5
<211>    20
<212>    DNA
<213>    Artificial Sequence

<220>
<223>    Forward primer for IL-10

<400>    5
ataactgcac ccacttccca                                                    20

<210>    6
<211>    20
<212>    DNA
<213>    Artificial Sequence

<220>
<223>    Reverse primer for IL-10

<400>    6
tcatttccga taaggcttgg                                                    20

<210>    7
<211>    21
<212>    DNA
<213>    Artificial Sequence

<220>
<223>    Forward primer for IL-17A

<400>    7
tttaactccc ttggcgcaaa a                                                  21

<210>    8
<211>    20
<212>    DNA
<213>    Artificial Sequence

<220>
<223>    Reverse primer for IL-17A

<400>    8
ctttccctcc gcattgacac                                                    20

<210>    9
<211>    19
<212>    DNA
<213>    Artificial Sequence

<220>
<223>    Forward primer for IL-1beta

<400>    9
caaccaacag atattctcc                                                     19

<210> 10
<211> 20
<212> DNA
<213> Artificial Sequence

<220>
<223> Reverse primer for IL-1beta

<400> 10
tgccgtcttt cattacacag 20

<210> 11
<211> 20
<212> DNA
<213> Artificial Sequence

<220>
<223> Forward primer for IL-12 p40

<400> 11
ggaacgacgg cagcagaata 20

<210> 12
<211> 24
<212> DNA
<213> Artificial Sequence

<220>
<223> Reverse primer for IL-12 p40

<400> 12
aacttgaggg agaagtagga atgg 24

<210> 13
<211> 21
<212> DNA
<213> Artificial Sequence

<220>
<223> Forward primer for IL-23 p19

<400> 13
tggcatcgag aaactgtgag a 21

<210> 14
<211> 25
<212> DNA
<213> Artificial Sequence

<220>
<223> Reverse primer for IL-23 p19

<400> 14
tcagttcgta ttggtagtcc tgtta 25

<210> 15

<212>   DNA
<213>   Artificial Sequence

<220>
<223>   Forward primer for NLRP3


<400>   15
agccttccag gatcctcttc                                                    20


<210>   16
<211>   20
<212>   DNA
<213>   Artificial Sequence

<220>
<223>   Reverse primer for NLRP3


<400>   16
cttgggcagc agtttctttc                                                    20


<210>   17
<211>   22
<212>   DNA
<213>   Artificial Sequence

<220>
<223>   Forward primer for TLR1


<400>   17
ccctacagaa acgtcctata cc                                                 22


<210>   18
<211>   22
<212>   DNA
<213>   Artificial Sequence

<220>
<223>   Reverse primer for TLR1


<400>   18
atgataagct cacattcctc ag                                                 22


<210>   19
<211>   20
<212>   DNA
<213>   Artificial Sequence

<220>
<223>   Forward primer for TLR2


<400>   19
gacaaagcgt caaatctcag                                                    20


<210>   20
<211>   20
<212>   DNA
<213>   Artificial Sequence

<220>
<223>    Reverse primer for TLR2

<400>    20
ccagaagcat cacatgacag                                                    20

<210>    21
<211>    22
<212>    DNA
<213>    Artificial Sequence

<220>
<223>    Forward primer for TLR3

<400>    21
taaagcgagt ttcactttca gg                                                 22

<210>    22
<211>    23
<212>    DNA
<213>    Artificial Sequence

<220>
<223>    Reverse primer for TLR3

<400>    22
gcagtttaac ttcccagata gag                                                23

<210>    23
<211>    20
<212>    DNA
<213>    Artificial Sequence

<220>
<223>    Forward primer for TLR4

<400>    23
ccctgcatag aggtagttcc                                                    20

<210>    24
<211>    20
<212>    DNA
<213>    Artificial Sequence

<220>
<223>    Reverse primer for TLR4

<400>    24
gtttgagagg tggtgtaagc                                                    20

<210>    25
<211>    20
<212>    DNA
<213>    Artificial Sequence

<220>
<223>    Forward primer for TLR5

<400>    25
caggatgttg gctggtttct                                                    20

<210>    26
<211>    20
<212>    DNA
<213>    Artificial Sequence

<220>
<223>    Reverse primer for TLR5

<400>    26
cggataaagc gtggagagtt                                                    20

<210>    27
<211>    20
<212>    DNA
<213>    Artificial Sequence

<220>
<223>    Forward primer for TLR6

<400>    27
tgctggaaat agagcttgga                                                    20

<210>    28
<211>    20
<212>    DNA
<213>    Artificial Sequence

<220>
<223>    Reverse primer for TLR6

<400>    28
ggacatgagt aaggttcctg                                                    20

<210>    29
<211>    21
<212>    DNA
<213>    Artificial Sequence

<220>
<223>    Forward primer for TLR7

<400>    29
caagaaagat gtccttggct c                                                  21

<210>    30
<211>    20
<212>    DNA
<213>    Artificial Sequence

<220>
<223>    Reverse primer for TLR7

<400>    30

ccatcgaaac ccaaagactc 20

<210> 31
<211> 20
<212> DNA
<213> Artificial Sequence

<220>
<223> Forward primer for TLR8

<400> 31
ttgccaaagt ctgctctctg 20

<210> 32
<211> 20
<212> DNA
<213> Artificial Sequence

<220>
<223> Reverse primer for TLR8

<400> 32
catttgggtg ctgttgtttg 20

<210> 33
<211> 19
<212> DNA
<213> Artificial Sequence

<220>
<223> Forward primer for TLR9

<400> 33
cccaacatgg ttctccgtc 19

<210> 34
<211> 21
<212> DNA
<213> Artificial Sequence

<220>
<223> Reverse primer for TLR9

<400> 34
gggtacagac ttcaggaaca g 21